# EUROPEAN PATENT SPECIFICATION

(11) **EP 4 676 542 B1**
(45) Date of publication and mention of the grant of the patent: **11.11.2026**
(21) Application number: 24712333.4
(22) Date of filing: 11.03.2024
(51) Int. Cl.: A61K 47/68, A61P 35/00

(54) **TRANS-CYCLOOCTENE WITH IMPROVED T-LINKER**
TRANS-CYCLOOCTEN MIT VERBESSERTEM T-LINKER
TRANS-CYCLOOCTÈNE AVEC UN LIEUR T AMÉLIORÉ

(30) Priority: 10.03.2023 EP 23161339; 13.03.2023 EP 23161604; 22.05.2023 EP 23174613; 13.12.2023 EP 23216318; 15.12.2023 EP 23217334
(43) Date of publication of application: 14.01.2026
(60) Divisional application: 26208177.1 / 4 837 638
(73) Proprietor: Tagworks Pharmaceuticals B.V., 6525 ED Nijmegen (NL)
(72) Inventor: ROSSIN, Raffaella, 6525 ED Nijmegen (NL); ROBILLARD, Marc Stefan, 6525 ED Nijmegen (NL); KLEIJN, Laurens Henri Johan, 6525 ED Nijmegen (NL); VERSTEEGEN, Ronny Mathieu, 6525 ED Nijmegen (NL)
(74) Representative: V.O.
(86) International application number: PCT/NL2024/050118
(87) International publication number: WO 2024/191293

(56) References cited:
- WO-A1-2019/212356
- WO-A1-2019/212357
- WO-A1-2020/123882
- WO-A1-2021/119268
- VAN DE GRAAFF MICHEL J. ET AL: "Conditionally Controlling Human TLR2 Activity via Trans-Cyclooctene Caged Ligands", vol. 31, no. 6, 8 June 2020 (2020-06-08), US, pages 1685 - 1692, XP055920398, ISSN: 1043-1802, Retrieved from the Internet <URL:http://pubs.acs.org/doi/pdf/10.1021/acs.bioconjchem.0c00237> [retrieved on 20220512], DOI: 10.1021/acs.bioconjchem.0c00237

## Description

### Technological field

The disclosure disclosed herein relates to trans-cyclooctenes (TCOs) with improved properties. Compositions and combinations comprising the TCOs of the disclosure, as well as methods for using same are provided as well.

### Background

In the field of bioorthogonal chemistry the ligation between TCOs and dienes, in particular tetrazines, has been studied in depth. While the ligation works well both *in vitro* as *in vivo,* identifying optimal compounds for clinical use remains a research focus.

Along these lines, it is desired to identify new TCOs with overall good *in vitro* and *in vivo* properties, *e.g.* one or more of: fast blood clearance rate, high uptake at the target site *(e.g.* a tumor), low off-target uptake, a good metabolism profile, good stability, good reactivity with tetrazines, and/or high payload release (especially *in vivo).*

There is thus a need for new TCOs that address one or more of the abovementioned problems and/or desires.

Background art includes WO 2019/212357; WO 2021/119268; WO 2019/212356; Van de Graaff et al., Bioconjugate Chemistry 2020, volume 31, issue 6, pages 1685-1692; and WO 2020/123882.

### Summary

The disclosure relates to at least the following embodiments:
Embodiment 1. A compound according to Formula (2): 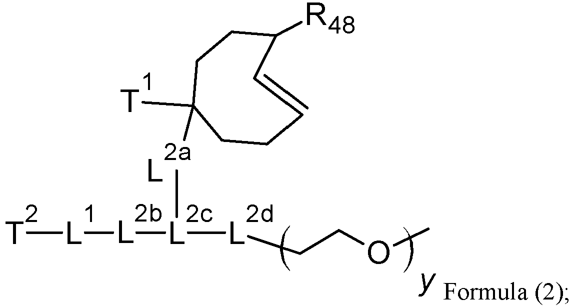 wherein
   **L¹ is** a linear C₅-C₆ alkylene;
   **T¹ is** -OH;
   **R₄₈** is -O-C(O)-C^{A}; wherein **C^{A}** is a drug, wherein the drug is selected from the group consisting of monomethyl auristatin E, exatecan, and N-methyl-exatecan;
   wherein the drug is linked to the moiety -O-C(O)- via a secondary or tertiary nitrogen atom that is part of the drug, forming a carbamate;
   **L²a, L^{2b},** and **L^{2d}** are each independently selected from the group consisting of -C(O)NH-, and -NHC(O)-;
   **L^{2c}** is C₄-C₆ alkanetriyl;
   **T²** is maleimidyl;
   y is an integer in a range of from 20 to 30.
Embodiment 2. The compound according to Embodiment 1, wherein L¹ is an unsubstituted, linear C₅ alkylene.
Embodiment 3. The compound according to any one of the preceding Embodiments, wherein T¹ is in an axial position, and wherein R₄₈ is in an axial position.
Embodiment 4. The compound according to any one of the preceding Embodiments, wherein the drug is monomethyl auristatin E (MMAE).
Embodiment 5. The compound according to any one of the preceding Embodiments, wherein L¹ is an unsubstituted, linear C₅ alkylene, y is 24, the drug is monomethyl auristatin E, T¹ is in an axial position, and R₄₈ is in an axial position.
Embodiment 6. The compound according to any one of the preceding Embodiments, wherein said compound is according to Formula (O): 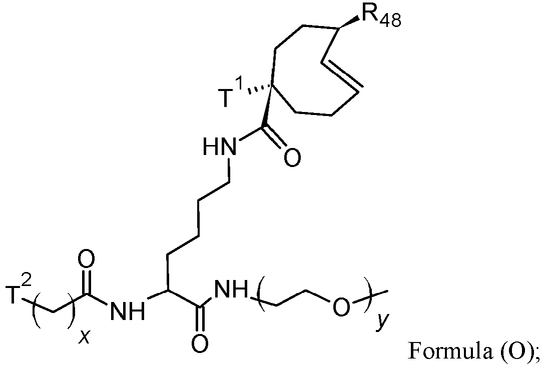 wherein x is 5, y is 24, the drug is monomethyl auristatin E, T¹ is in an axial position, and R₄₈ is in an axial position.
Embodiment 7. The compound according to any one of the preceding Embodiments, wherein said compound is: 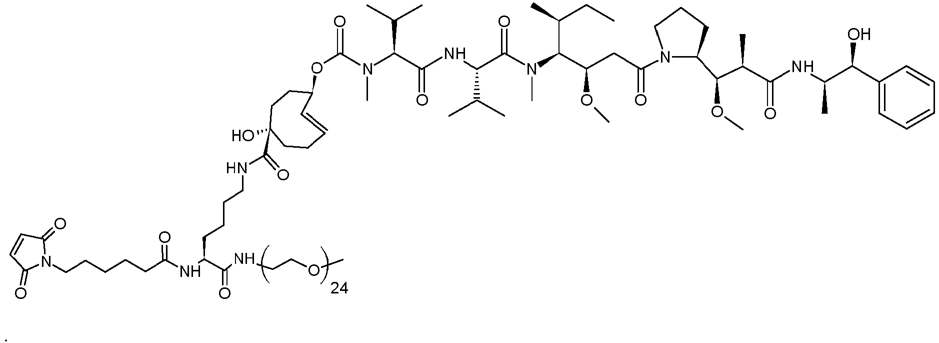
Embodiment 8. The compound according to any one of the preceding Embodiments, wherein said compound is 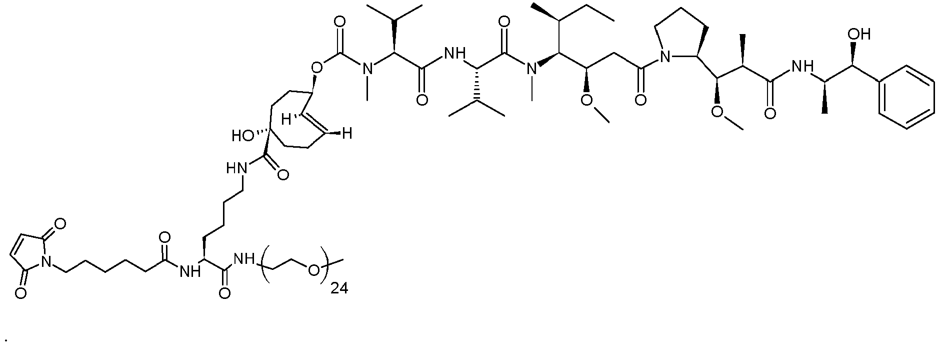
Embodiment 9. A conjugate comprising a protein conjugated to at least one compound according to any one of the preceding Embodiments, wherein said protein and said compound are conjugated via T² and a residue of a sulfhydryl of said protein; wherein the protein is AVP0458 consisting of two monomers, wherein each of the two monomers has an amino acid sequence according to SEQ ID NO: 1; and wherein the protein is conjugated to at most 4 of said compounds.
Embodiment 10. The conjugate according to Embodiment 9, wherein the at least one compound is according to Embodiment 5.
Embodiment 11. The conjugate according to Embodiment 9, wherein the conjugate is 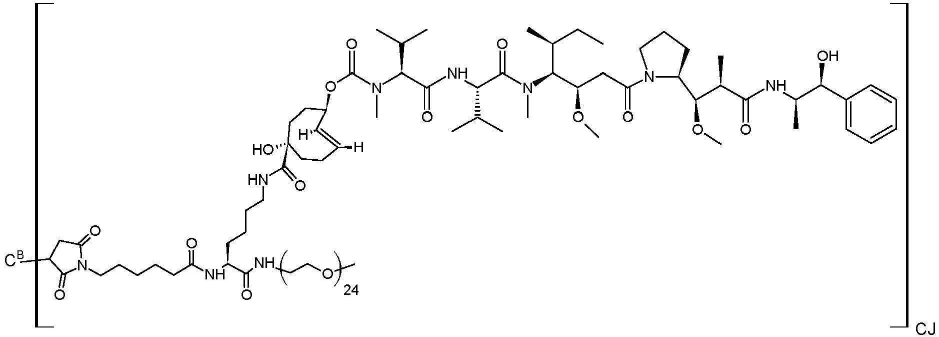 wherein C^{B} is AVP0458; and CJ is of from 3.5 to 4.
Embodiment 12. A composition comprising:
   (a) a compound according to any one of Embodiments 1 to 8; and/or
   (b) the conjugate according to any one of Embodiments 9 to 11;
      preferably the composition is a pharmaceutical composition.
Embodiment 13. A composition according to Embodiment 12, wherein said composition comprises:
   (a) a compound according to any one of Embodiments 1 to 6; and
   (b) the enantiomer of said compound;
      preferably said composition is a racemic mixture of (a) and (b).
Embodiment 14. A combination of
   (A1) a compound according to any one of Embodiments 1 to 8;
   (A2) a conjugate according to any one of Embodiments 9 to 11; and/or
   (A3) a composition according to Embodiment 12 or 13; with
   (B) a diene; preferably the diene is a tetrazine.
Embodiment 15. The combination according to Embodiment 14, wherein the diene is selected from the group consisting of: 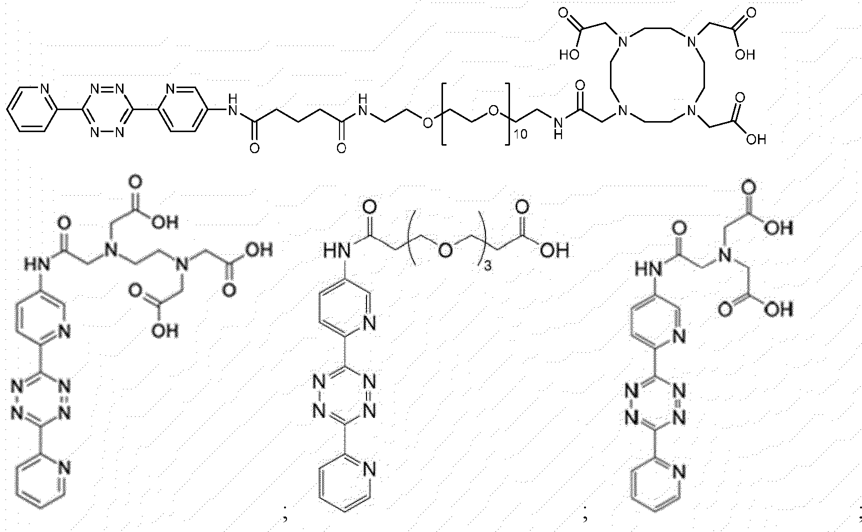 and 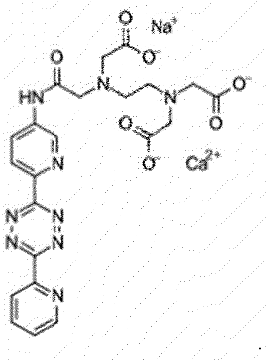
Embodiment 16. The compound according to any one of Embodiments 1 to 8; the conjugate according to any one of Embodiments 9 to 11; the composition according to any one of Embodiments 12 to 13; or the combination according to any one of Embodiments 14 to 15; for use as a medicament.
Embodiment 17. The compound according to any one of Embodiments 1 to 8; the conjugate according to any one of Embodiments 9 to 11; the composition according to any one of Embodiments 12 to 13; or the combination according to any one of Embodiments 14 to 15; for use in the treatment of a disease in a subject; wherein the subject is a human; and the disease is cancer.
Embodiment 18. A non-therapeutic method for reacting:
   (ia) the compound according to any one of Embodiments 1 to 8;
   (iia) the conjugate according to any one of Embodiments 9 to 11; and/or
   (iiia) the composition according to any one of Embodiments 12 to 13;
   with a diene,
   wherein said method comprises the step of contacting (ia), (iia), or (iiia) with said diene, preferably said non-therapeutic method is an *in vitro* method; and preferably said diene is a tetrazine.
Embodiment 19. A non-therapeutic use of:
   (a) the compound according to any one of Embodiments 1 to 8;
   (b) the conjugate according to any one of Embodiments 9 to 11;
   (c) the composition according to any one of Embodiments 12 to 13; and/or
   (d) the combination according to any one of Embodiments 14 to 15; in a click reaction.
Embodiment 20. A method for synthesizing a compound according to any one of Embodiments 1 to 8; wherein said method comprises
   (A) coupling a compound of Formula (R) to a compound of Formula (S): 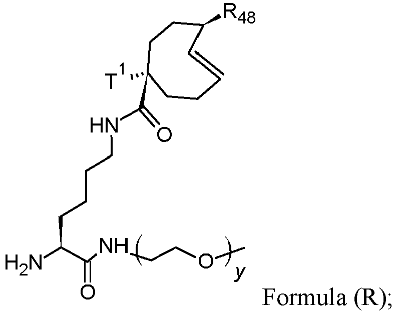 wherein R₄₈, T¹, and y are as defined in any one of Embodiments 1 to 8; 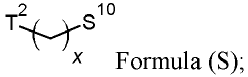 wherein T², and x, are as defined in any one of Embodiments 1 to 8, and S¹⁰ is -COOH or an active ester, preferably S¹⁰ is -COOH;
      or
   (B) coupling a compound of Formula (T) to a compound of Formula (U): 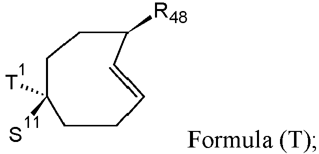 wherein R₄₈, and T¹ are as defined in any one of Embodiments 1 to 8; and S¹¹ is -COOH or an active ester, preferably S¹¹ is an active ester; 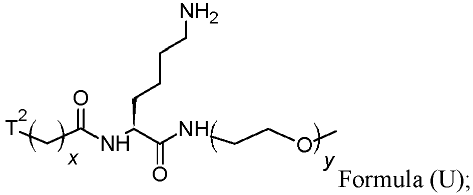 wherein T², x, and y are as defined in any one of Embodiments 1 to 8.
Embodiment 21. A method for synthesizing a conjugate according to any one of Embodiments 9 to 11; wherein said method comprises the step of coupling a protein to a compound according to any one of Embodiments 1 to 8; wherein preferably in said protein disulfide bonds have been reduced.

### Detailed Description

As an example from the field of bioorthogonal chemistry, WO 2022/197182 describes AVP0458-22-PEG24, herein referred to as compound 1. Compound 1 is an AVP0458 diabody modified with four TCO-containing moieties, and has the following structure:

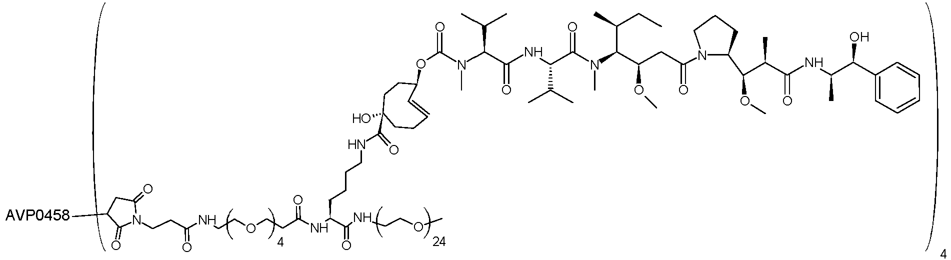

However, the inventors have identified, for the first time, that certain properties of compound 1 may be improved.

First, it was found that while compound 1 has a good clearance from blood, further improvements are desired. Compound 1 has a half-life in the blood of healthy mice of 4.22 hours, and 48 hours after injection in healthy mice 1.14% ID/g of compound 1 in the blood of said mice was observed. Based on this, it is desired that new TCOs be provided that show faster clearance rates as compared to compound 1.

Second, it was found that while compound 1 shows good tumor uptake of 18.42 %ID/g in LS174T xenograft bearing mice, further improvements are desired. It is therefore also desired that TCOs with a higher tumor uptake be provided.

Third, it was observed that compound 1 shows uptake at off-target sites, *e.g.* non-tumour sites such as the heart, lung, etc. It is also desired that TCOs be provided with a lower off-target uptake.

Fourth, it was observed that compound 1 shows a metabolism profile that can be improved. Thus, it is also desired that TCOs be provided showing a better metabolism profile.

Some aspects and embodiments of the disclosure are therefore, in a broad sense, based on the judicious insight that TCOs of the disclosure may meet one or more of the aforementioned desires. In particular, it was surprisingly found that replacing the PEG4 moiety of compound 1 may result in a higher clearance rate, higher tumor uptake, lower off-target uptake, a better metabolism profile, and/or further improved *in vitro* and *in vivo* properties.

Especially the combination of a faster clearance rate and a higher tumor uptake is surprising, since this means that the faster clearance from blood is not due to e.g. excretion from the body. Instead, the compounds of the disclosure may be quickly taken up in the tumour. Even more advantageously the uptake of compounds of the disclosure in off-target tissues may be much lower as compared to the uptake in the tumour. This means that a higher percentage of payload may be released at the desired target site, and that the trigger to activate this payload release (typically a diene, *e.g.* a tetrazine) may be administered at an earlier point in time, shortening the entire procedure. Thus, the compounds of the disclosure may also result in a higher convenience for patients, as fewer and/or less severe side-effects may be expected as well as a shorter treatment time.

Preferred embodiments of the disclosure are further described below, also in relation to a List of Clauses below. All of these embodiments, regardless of whether said embodiments are disclosed in the general part of the description or as part of the List of Clauses, can be combined as long as said embodiments are not mutually exclusive.

### Compounds of the disclosure

It is understood that any compounds as provided herein may be in a form, formulation or solution in which the compound is present as a salt, solvate or hydrate of the compound. Accordingly, whereever herein a compound or genus of compounds are provided, or reference is made to "compound of the disclosure" or "compounds of the disclosure", it will be understood that also the salt, hydrate, or solvate of said compound(s) are included by such a statement even if the terms salt, hydrate or solvate are not specifically mentioned in each instance. In certain embodiments, a compound of the disclosure is purified or in a form or state in which it is not present as a salt, or as a hydrate, or as a solvate of the compound; however, unless specifically indicated as such it is intended to be assumed that any compound herein may be in the form of a salt, hydrate or solvate.

Preferred embodiments of the compounds of the disclosure are further described below in relation to several Formulae and variables.

### Formulae

Preferably, the compound of the disclosure is according to a Formula selected from the group consisting of Formula (2), Formula (3), Formula (O), Formula (P), and Formula (Q).

Even more preferably, the compound of the disclosure is according to Formula (O). Yet more preferably, the compound of the disclosure is according to Formula (3). Still more preferably, the compound of the disclosure is according to Formula (P). Even more preferably, the compound of the disclosure is according to Formula (Q). Also disclosed herein but not according to the invention are compounds according to Formula (L), Formula (M) and Formula (N).

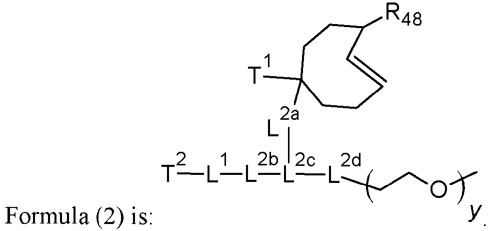

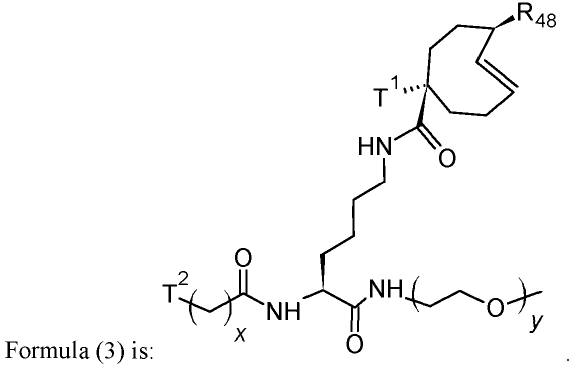

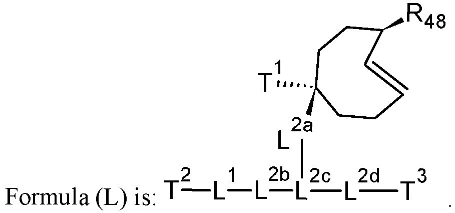

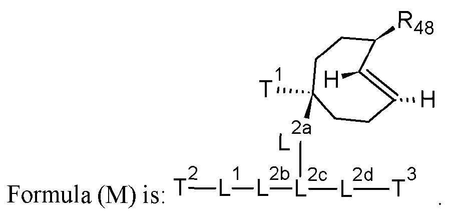

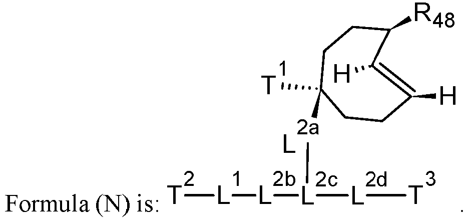

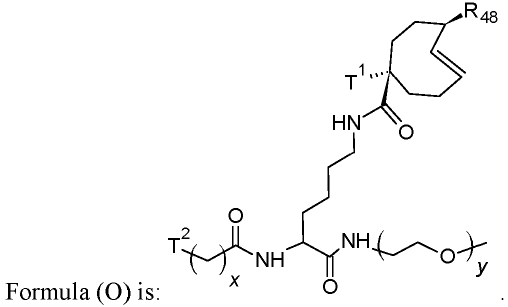

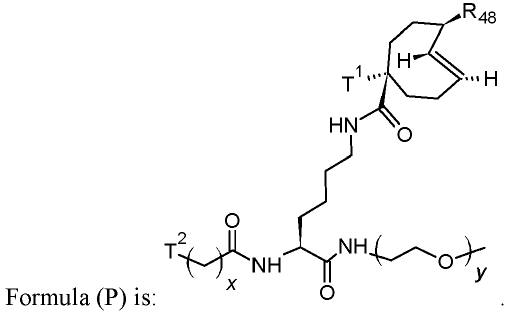

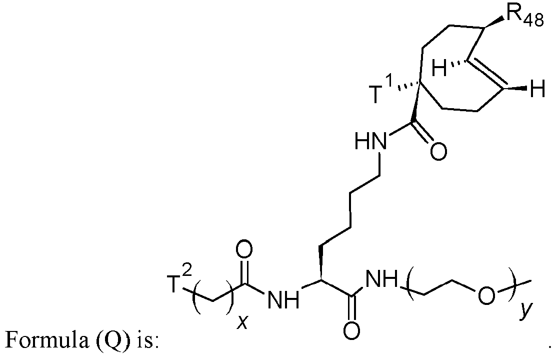

### L¹

L¹ is a linear C₅-C₆ alkylene. Most preferably, L¹ is a linear C₅ alkylene.

L¹ can be substituted or unsubistuted. Preferably, L¹ is unsubstituted. Most preferably, L¹ is an unsubstituted, linear C₅ alkylene.

Without wishing to be bound by theory, the inventors believe that the linker L¹ of compounds of Formula (1) of the present disclosure may provide a faster blood clearance rate, while maintaining a high uptake at the target site of the compound of Formula (1). Still without wishing to be bound by theory, an advantage of linkers L¹ having a length as defined in claim 1 may be that sufficient distance between the moiety C^{B} and the trans-cyclooctene can be achieved, so that the double bond of the trans-cyclooctene may readily react with a diene. Still without wishing to be bound by theory, an advantage of linkers L¹ having a length as defined in claim 1 may be that they are not too long, so that they may still be shielded by moiety C^{B} which may prevent e.g. deactivation. An advantage of relatively short alkylene linkers, such as C₄-C₆ alkylene, may be that their solubility is also higher than for relatively long alkylene linkers.

### L²

L² has the following structure:

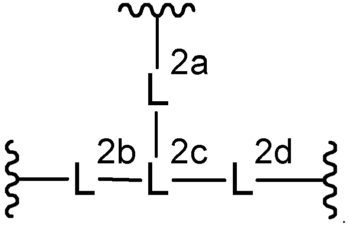

More preferably, L² is:

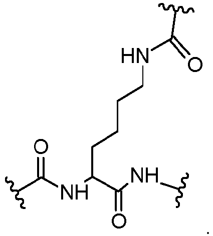

Even more preferably, L² is:

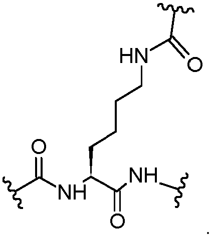

More preferably still, L² is:

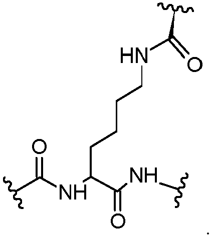

Most preferably, L² is:

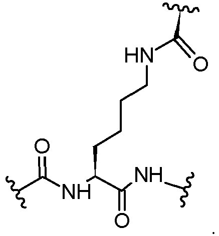

### L²a, L^{2b}, L^{2c}, and L^{2d}

Most preferably, L^{2a} is -NHC(O)-. Most preferably, L^{2b} is -NHC(O)-.

More preferably, L^{2c} is C₄-C₅ alkanetriyl. More preferably than the foregoing, L^{2c} is C₅ alkanetriyl. Most preferably, L^{2c} is >CH-CH₂-CH₂-CH₂-CH₂-.

Most preferably, L^{2d} is -C(O)NH-.

### T¹

Preferably, T¹ is in an axial position. Without wishing to be bound by theory, the inventors believe that in that case and when R₄₈ is a releasable group, when the compound of the disclosure reacts with a diene, T¹ aids in releasing the payload. This results in optimal release yields and/or release kinetics.

### T²

More preferably, T² is N-maleimidyl. In these embodiments, it is most preferred that T² is:

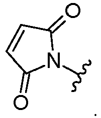

In other embodiments, T² is a residue of a bioconjugation moiety. These embodiments typically relate to conjugates of the disclosure, wherein T² links to *e.g.* a protein. Such residues are well-known to the skilled person. In these embodiments, it is most preferred that T² is: 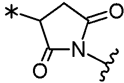 wherein the asterisk indicates a bond to the protein, and the wiggly line denotes a bond to the rest of the compound of the disclosure.

### C^{B}

C^{B} is AVP0458 consisting of two monomers, wherein each of the two monomers has an amino acid sequence according to SEQ ID NO: 1.

Preferably, C^{B} is linked to the remainder of the compound of the disclosure or the conjugate of the disclosure via S or N that is part of C^{B}. More preferably, C^{B} is linked to the remainder of the compound of the disclosure or the conjugate of the disclosure via S that is part of C^{B}.

### AVP0458

As used herein, AVP0458 refers to a TAG72-binding diabody derived from the CC49 antibody. AVP0458 is a diabody consisting of two monomers, each monomer having an amino acid sequence according to SEQ ID NO: 1: 
**SEQ ID NO:1** (amino acid sequence of AVP0458 diabody monomer):
SVQLQQSDAELVKPGASVKISCKASGYTFTDHAIHWVKQNPEQGLEWIGYFSPGNDD
FKYNERFKGKATLTADKSSSTAYLQLNSLTSEDSAVYFCTRSLNMAYWGQGTSVTV
SSGGGGSDIVMTQSCSSCPVSVGEKVTLSCKSSQSLLYSGNQKNYLAWYQQKPGQSP
KLLIYWASTRESGVPDRFTGSGSGTDFTLSISSVETEDLAVYYCQQYYSYPLTFGAGT
KLVLKR
 Herein, the underlining indicates the cysteines that are preferably modified with or linked to a compound of the disclosure or the remainder thereof if AVP0458 is itself part of the compound of the disclosure.

Thus, in SEQ ID NO: 1 it is preferred that at least one of the underligned cysteines, more preferably both underlined cysteines, is modified with or linked to a compound according to the disclosure. In other words: it is preferred that the sulfur atom of the underlined cysteines is coupled to a moiety T² as defined herein, preferably T² is the residue of an N-maleimidyl group.

### Variable x in Formulae (3), (O), (P), and (Q)

In Formula (3), Formula (O), Formula (P), and Formula (Q), x is an integer in a range of from 4 to 12; preferably x is an integer in a range of from 4 to 8, more preferably x is an integer in a range of from 4 to 6, and most preferably x is 5.

### R₄₈

Preferably, R₄₈ is in the axial position. This preference holds especially when R₄₈ is a releasable group. Having the releasable group in an axial position results in better release of the payload as compared to having the releasable group in an equatorial position. Group R₄₈ is a releasable group.

The releasable group is -O-C(=O)-C^{A}. C^{A} is Construct A, which is a payload. Most preferably, C^{A} is monomethyl auristatin E (MMAE).

Preferably, C^{A} is linked to the moiety -O-C(=O)- via a secondary or tertiary nitrogen atom that is part of C^{A}, forming a carbamate. Preferably, C^{A} is monomethyl auristatin E (MMAE) linked to the moiety -O-C(=O)- via a secondary or tertiary nitrogen atom that is part of MMAE, forming a carbamate; or C^{A} is exatecan or N-methyl-exatecan linked to the moiety -O-C(=O)-, via a primary or secondary nitrogen atom that is part of exatecan, forming a carbamate. More preferably, C^{A} is monomethyl auristatin E (MMAE) linked to the moiety -O-C(=O)-, via a secondary or tertiary nitrogen atom that is part of MMAE, forming a carbamate.

Preferably, group R₄₈ is: 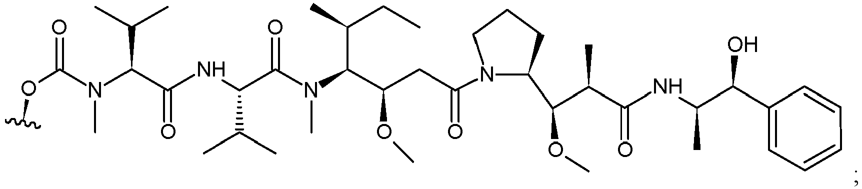 or 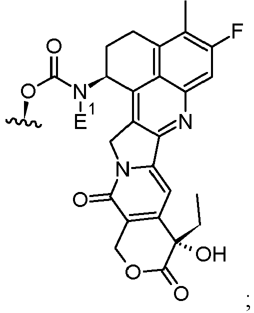 wherein E¹ is -H or -CH₃, preferably E¹ is -H.

Most preferably, group R₄₈ is:

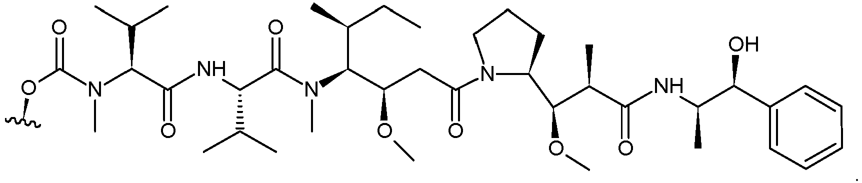

### Log P

In preferred embodiments the Log P of compounds of Formula (1) have a value in a range of from 2.0 and -2.0, more preferably in a range of from 1.0 and -1.0.

In embodiments where it is required that a compound as disclosed herein, in particular a diene, has an extracellular volume of distribution it is preferred that the Log P of said compound is at most 2, preferably at most 1, more preferably at most 0, even more preferably at most -1. In embodiments where it is required that a compound as disclosed herein, in particular a diene, has an intracellular volume of distribution it is preferred that the Log P of the Activator is at least -1, preferably at least 0, more preferably at least 1, even more preferably at least 2.

### Molecular weight

For a compound of Formula (1) wherein T² is a bioconjugation moiety, it is preferred that the molecular weight of said compound is at most 5 kDa, more preferably at most 4 kDa, even more preferably at most 3.5 kDa, more preferably stil at most 3 kDa, and most preferably at most 2.5 kDa.

### Conjugates of the disclosure

The disclosure also relates to a conjugate, or a salt, hydrate, or solvate thereof, wherein the conjugate comprises a protein conjugated to at least one compound according to the disclosure wherein T² is a residue of a bioconjugation moiety, and said protein and said compound are conjugated via T². Thus, the conjugate of the disclosure is to be understood as a compound of the disclosure (wherein T² was originally a bioconjugation moiety) linked to a protein via T², wherein due to the coupling of said compound and said protein, T² in the conjugate of the disclosure is the residue of an N-maleimidyl group, viz.: 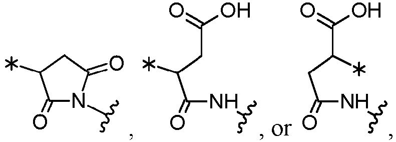 wherein the asterisk indicates a bond to the protein, and the wiggly line denotes a bond to the rest of the compound of the disclosure.

In the conjugate of the disclosure, the protein is AVP0458 consisting of two monomers, wherein each of the two monomers has an amino acid sequence according to SEQ ID NO: 1.

In the conjugate of the disclosure the protein and the compound of the disclosure are conjugated via T² and a residue of a sulfhydryl of said protein. Preferably, the residue of the sulfhydryl group of said protein is part of a cysteine residue of said protein.

Preferably, the conjugate of the disclosure is 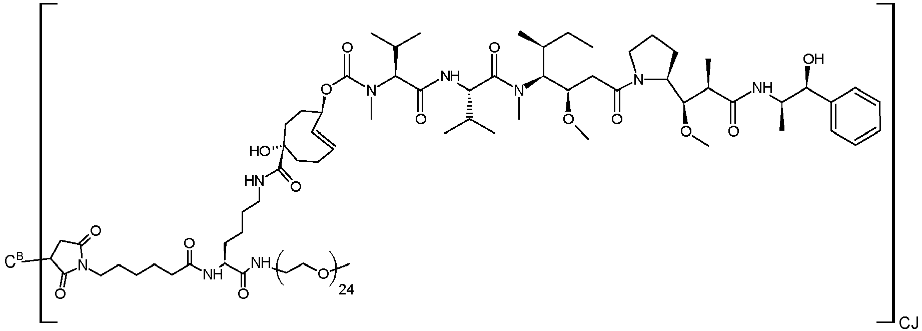 or 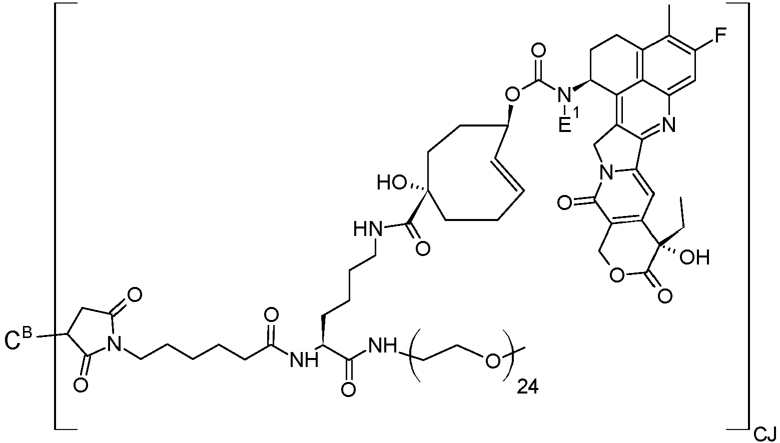 wherein E¹ is -H or -CH₃, preferably E¹ is -H.

More preferably, the conjugate of the disclosure is

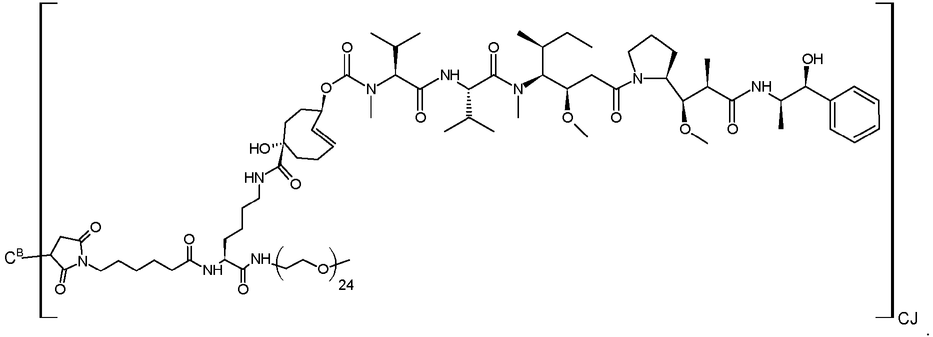

In relation to conjugates of the disclosure, CJ is in a range of from 3.5 to 4. It will be understood that for individual conjugates, CJ is typically an integer, and is most preferably about 4. When measuring CJ for multiple conjugates, however, an average number may be obtained, which is not necessarily an integer. As CJ is commonly determined for multiple conjugates, CJ in relation to the disclosure typically refers to an average number.

More preferably, the conjugate is: 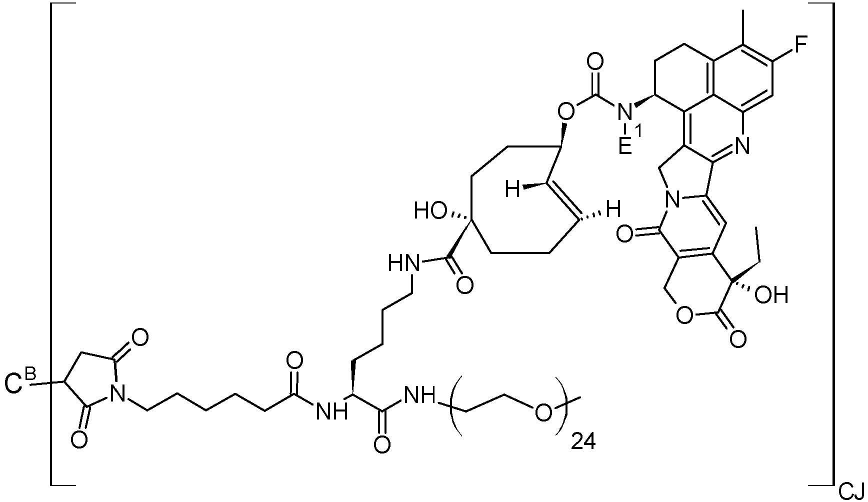 wherein E¹ is -H or -CH₃, preferably E¹ is -H.

More preferably, the conjugate is: 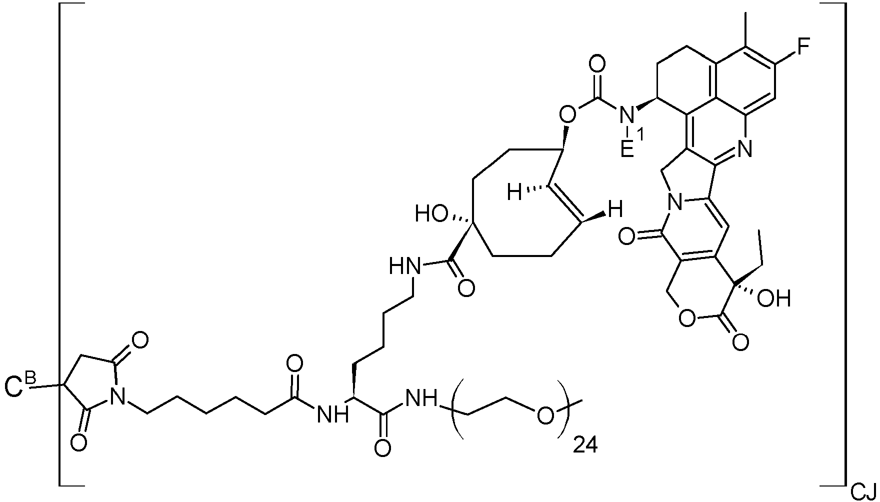 wherein E¹ is -H or -CH₃, preferably E¹ is -H.

More preferably, the conjugate is:

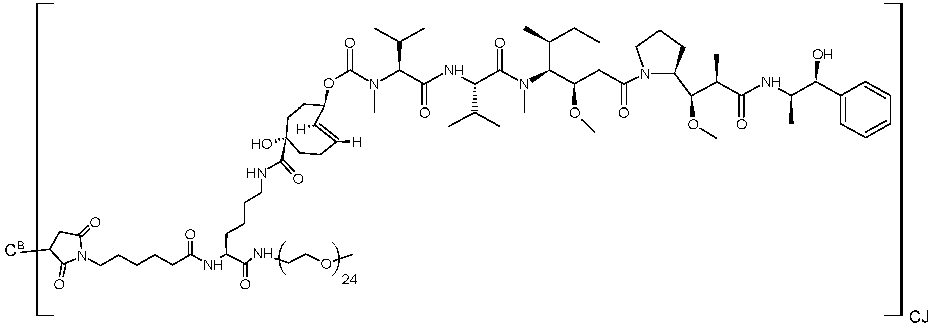

More preferably, the conjugate is:

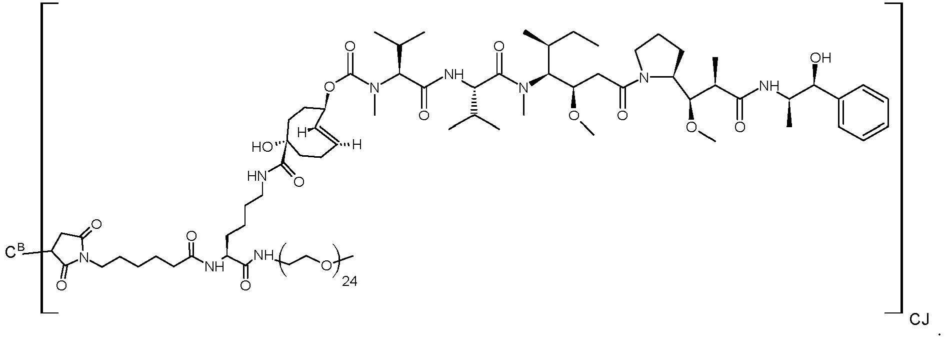

### Compositions of the disclosure

The disclosure also pertains to a composition comprising a compound according to the disclosure, or the salt, hydrate, or solvate thereof. Preferably, the composition is a pharmaceutical composition. Preferably, the composition of the disclosure further comprises a pharmaceutically acceptable carrier. It is also preferred that if a salt of a compound of the disclosure is included in the composition of the disclosure, a pharmaceutically acceptable salt is used.

### Combinations of the disclosure

The disclosure also relates to a combination of (A1) a compound according to the disclosure, or the salt, hydrate, or solvate thereof; (A2) a conjugate according to the disclosure, or the salt, hydrate, or solvate thereof; and/or (A3) a composition according to the disclosure; with (B) a diene or a salt, solvate, or hydrate thereof. It will be understood that herein, a compound according to the disclosure is a dienophile and/or comprises a dienophile moiety, and may be called a "Trigger". The diene may be referred to as an "Activator".

Preferably, the combination is of (A1) and (B). Preferably, the combination is of (A2) and (B). Preferably, the combination is of (A3) and (B). Preferably, the combination is of (A1), (A2), and (B). Preferably, the combination is of (A1), (A3), and (B). Preferably, the combination is of (A2), (A3), and (B). Preferably, the combination is of (A1), (A2), (A3), and (B).

Preferably, the combination of the disclosure is a kit. More preferably, the combination of the disclosure is a kit wherein (A1), (A2), and/or (A3) is/are physically separated from (B).

Preferably the diene is a tetrazine. More preferably, the diene is selected from the group consisting of: 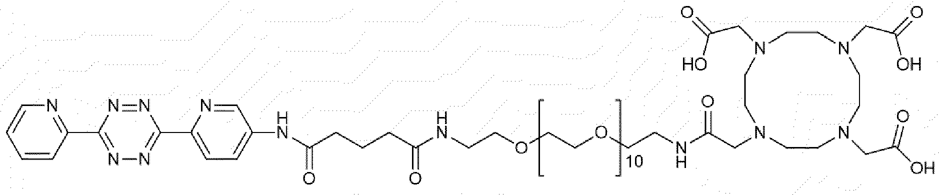 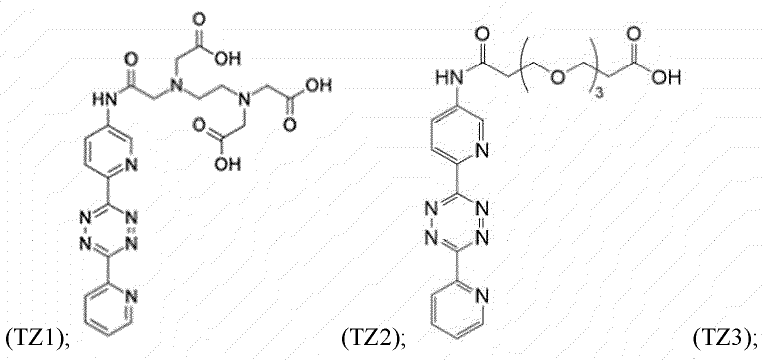 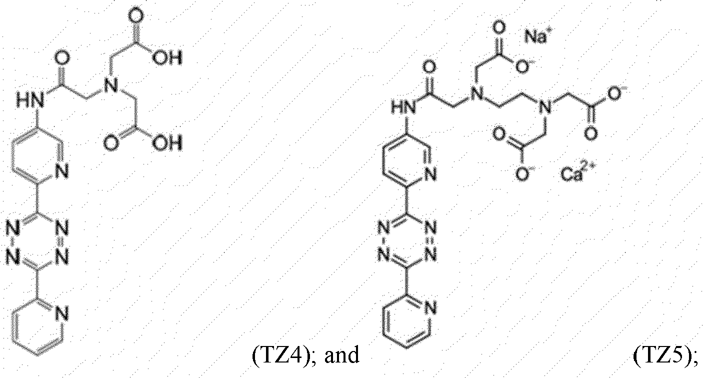 or a salt, hydrate, and/or solvate thereof.

Preferably, the diene is (TZ1) or a salt, hydrate, and/or solvate thereof. More preferably, the diene is (TZ2) or a salt, hydrate, and/or solvate thereof. More preferably, the diene is (TZ3) or a salt, hydrate, and/or solvate thereof. More preferably, the diene is (TZ4) or a salt, hydrate, and/or solvate thereof. Most preferably, the diene is (TZ5) or a salt, hydrate, and/or solvate thereof.

(TZ1) is the best-studied tetrazine for *in vivo* use in literature, and the most promising candidate for clinical use. Reference is made to, *inter alia,* Rossin et al., Angew. Chem. Int. Ed. 2010, volume 49, pages 3375-3378; Rossin et al., J. Nucl. Med. 2013, volume 54; pages 1989-1995; Rossin et al., Bioconjugate Chem. 2013, volume 24, pages 1210-1217; Rossin et al., Mol. Pharm. 2014, volume 11, pages 3090-3096; Van Duijnhoven et al., J. Nucl. Med. 2015, volume 56, pages 1422-1428; Edem et al. Molecules 2020, volume 25, page 463; Rossin et al., Bioconjugate Chem. 2016, volume 27, pages 1697-1706; Rossin et al., Nature Commun. 2018, volume 9, article 1484; WO 2020/256546 (in particular Example 5 at pages 294-296).

However, the inventors have identified several hitherto unknown disadvantages of (TZ1). These problems mainly arise when using (TZ1) *in vivo* as an activator for the payload release from an eight-membered non-aromatic cyclic mono-alkenylene moiety (such as a trans-cyclooctene), which require higher doses than when using (TZ 1) for radioimaging and/or radiotherapy.

First, it was found that compound (TZ1) strongly inhibits the physiologically relevant enzymes cyclooxygenase (COX-1), acetyl cholinesterase (ACES), monoamine oxidase (MAO-B), and calcium channel L-type, dihydropyridine. Each of these proteins is important in maintaining health in a subject, and undesired inhibition of these enzymes and/or transporter may lead to side-effects.

Second, it was found that (TZ1) has a relatively low maximum tolerated dose (MTD) in mice of about 39 µmol/kg.

Furthermore, the synthesis of (TZ1) comprises many steps, while it is preferred that tetrazines are used that can be synthesized in fewer steps.

Finally, it is desired that tetrazines with overall good *in vitro* and *in vivo* properties be provided, *i.e.* one or more of: good stability, good reactivity with and/or high payload release from trans-cyclooctenes (especially *in vivo),* low membrane permeability, low cell toxicity, and low genotoxicity.

It was found that (TZ2), (TZ3), (TZ4), and in particular (TZ5) overcome one or more of these disadvantages of (TZ1). Therefore, combinations with at least one of (TZ2), (TZ3), (TZ4), and (TZ5) are preferred over combinations comprising (TZ1), and combinations with (TZ5) are most preferred.

### Non-therapeutic methods using and uses for using compounds of the disclosure

**In** some embodiments, the disclosure pertains to non-therapeutic methods and non-therapeutic uses. Preferably, the dienophile used therein is as described in relation to the combination of the disclosure.

For the non-therapeutic method of the disclosure it is preferred that the compound of the disclosure (viz. (ia)), the conjugate of the disclosure (viz. (iia)), and/or the composition of the disclosure (viz. (iiia)), and the diene are further contacted with a solvent. The skilled person is aware of suitable solvents for a reaction between a trans-cyclooctene (TCO) and a tetrazine. Preferably, the solvent comprises water, and more preferably the solvent is water.

For the non-therapeutic use, the click reaction is preferably a bioorthogonal click reaction. Preferably, the click reaction is performed *in vitro,* although non-therapeutic reactions *in vivo* can be carried out as well.

### Medical use

The disclosure also relates to a compound of the disclosure, or the salt, hydrate, or solvate thereof; the conjugate of the disclosure, or the salt, hydrate, or solvate thereof; the composition of the disclosure; or the combination of the disclosure; for use in the treatment of a disease in a subject.

In relation to the medical use, preferably the subject is a human. Preferably, the disease is cancer.

### Methods of synthesizing compounds of the disclosure

The disclosure also relates to a method for synthesizing a compound of the disclosure, wherein said method comprises coupling a compound of Formula (R) to a compound of Formula (S): 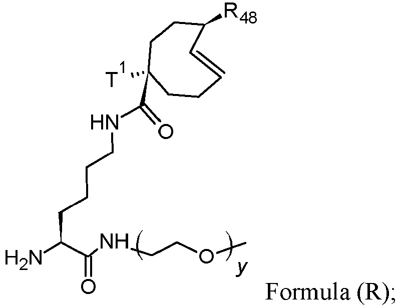 wherein R₄₈, T¹, and y are as defined herein; 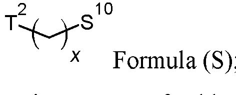 wherein T², and x, are as defined herein, and S¹⁰ is -COOH or an active ester, preferably S¹⁰ is -COOH. Preferably, in Formula (S) x is an integer of from 4 to 6, and most preferably x is 5.

In the method for synthesizing a compound of the disclosure, when S¹⁰ is -COOH, it is preferred that the compound of Formula (S) is contacted with at least one coupling reagent, preferably in the presence of a base, preferably a non-nucleophilic base. Preferred non-nucleophilic bases are N,N-diisopropylethylamine (DIPEA), 1,8-diazabicycloundec-7-ene (DBU), and 1,5-diazabicyclo(4.3.0)non-5-ene (DBN). Preferably, the at least one coupling reagent is selected from the group consisting of dicyclohexylcarbodiimide (DCC), diisopropylcarbodiimide (DIC), ethyl-(N',N'-dimethylamino)propylcarbodiimide hydrochloride (EDC), 1- hydroxybenzotriazole (HOBt), 4-(N,N-dimethylamino)pyridine (DMAP), (benzotriazol-1-yloxy)tris(dimethylamino)phosphonium hexafluorophosphate (BOP), (benzotriazol-1-yloxy)tripyrrolidinophosphonium hexafluorophosphate, (7-azabenzotriazol-1-yloxy)tripyrrolidinophosphonium hexafluorophosphate (PyAOP), bromotripyrrolidinophosphonium hexafluorophosphate (PyBrOP), O-(Benzotriazol-1-yl)-N,N,N',N'-tetramethyluronium hexafluorophosphate (HBTU), O-(Benzotriazol-1-yl)-N,N,N',N'-tetramethyluronium tetrafluoroborate (TBTU), O-(7-Azabenzotriazol-1-yl)-N,N,N',N'-tetramethyluronium hexafluorophosphate (HATU), O-(7-Azabenzotriazol-1-yl)- N,N,N',N'-tetramethyluronium tetrafluoroborate (TATU), O-(6-Chlorobenzotriazol-1-yl)-N,N,N',N'-tetramethyluronium hexafluorophosphate (HCTU), O-[(Ethoxycarbonyl)cyanomethylenamino]-N,N,N',N'-tetramethyluronium tetrafluoroborate (TOTU), (1-Cyano-2-ethoxy-2-oxoethylidenaminooxy)dimethylamino-morpholino-carbenium hexafluorophosphate (COMU), O-(N-Succinimidyl)-1,1,3,3-tetramethyl-uronium tetrafluoroborate (TSTU), O-(5-Norbornene-2,3-dicarboximido)-N,N,N',N'-tetramethyluronium tetrafluoroborate (TNTU), O-(1,2-Dihydro-2-oxo-1-pyridyl-N,N,N',N'-tetramethyluronium tetrafluoroborate (TPTU), N,N,N',N'-Tetramethyl-O-(3,4-dihydro-4-oxo-1,2,3-benzotriazin-3-yl)uronium tetrafluoroborate (TDBTU), 3-(Diethylphosphoryloxy)-1,2,3-benzotriazin-4(3H)-one (DEPBT), carbonyldiimidazole (CDI), N,N,N',N'-tetramethylchloroform-amidinium hexafluorophosphate (TCFH), thionyl chloride, oxalyl chloride, cyanuric chloride, cyanuric fluoride, phosphorous trichloride, phosphorous pentachloride, N-hydroxysuccinimide, N-hydroxysulfosuccinimide, and combinations thereof.

The skilled person is aware of suitable conditions to carry out a coupling reaction between a compound of Formula (R) and a compound of Formula (S).

Preferably, the coupling is carried out at a temperature of from -20°C to 80°C, more preferably of from 0°C to 60°C, even more preferably of from 4°C to 50°C, more preferably still of from 10°C to 40°C, and most preferably of from 15°C to 30°C.

Preferably, the coupling is carried out in the presence of a solvent, wherein preferably the solvent is an organic solvent.

The disclosure also relates to an alternative method for synthesizing a compound of the disclosure, wherein said method comprises coupling a compound of Formula (T) to a compound of Formula (U): 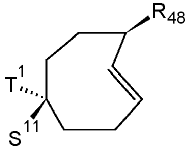 Formula (T); wherein T¹ and R₄₈ are as defined herein; and S¹¹ is -COOH or an active ester, preferably S¹¹ is an active ester, more preferably S¹¹ is selected from the group consisting of - C(O)O-N-succinimidyl, -C(O)O-pentafluorophenyl, -C(O)O-tetrafluorophenyl, -C(O)O-4-nitrophenyl, and -C(O)Cl; even more preferably, S¹¹ is -C(O)O-N-succinimidyl, or -C(O)O-pentafluorophenyl; and most preferably, S¹¹ is -C(O)O-pentafluorophenyl. 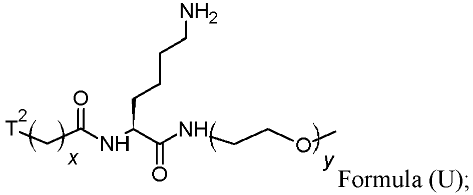 wherein T², x, and y, are as defined herein. Preferably, in Formula (U) x is an integer of from 4 to 6, and most preferably x is 5.

In the alternative method for synthesizing a compound of the disclosure, when S¹¹ is - COOH, it is preferred that the compound of Formula (S) is contacted with at least one coupling reagent, preferably in the presence of a base, preferably a non-nucleophilic base. Preferred non-nucleophilic bases are N,N-diisopropylethylamine (DIPEA), 1,8-diazabicycloundec-7-ene (DBU), and 1,5-diazabicyclo(4.3.0)non-5-ene (DBN). Preferably, the at least one coupling reagent is as defined above.

The skilled person is aware of suitable conditions to carry out a coupling reaction between a compound of Formula (T) and a compound of Formula (U). Preferably, the coupling is carried out at a temperature of from -20°C to 80°C, more preferably of from 0°C to 60°C, even more preferably of from 4°C to 50°C, more preferably still of from 10°C to 40°C, and most preferably of from 15°C to 30°C. Preferably, the coupling is carried out in the presence of a solvent, wherein preferably the solvent is an organic solvent.

### Methods of synthesizing conjugates of the disclosure

The disclosure also pertains to a method for synthesizing a conjugate of the disclosure, wherein said method comprises the step of coupling a protein to a compound of the disclosure, or a salt, hydrate, or solvate thereof; wherein in said compound T² is a bioconjugation moiety; wherein preferably in said protein disulfide bonds have been reduced.

As T² in the compound of the disclosure is a bioconjugation moiety that can react with a sulfhydryl group, namely an N-maleimidyl group, it is preferred that the protein contains free sulfhydryl groups. Typically, such sulfhydryl groups can be obtained by reducing disulfide bonds present in the protein. To that end, it is preferred that the protein has been contacted with a reducing agent prior to the coupling. Preferably, the reducing agent is selected from the group consisting of dithiothreitol (DTT), and tris-2-carboxyethylphosphine hydrochloride (TCEP).

If the protein is contacted with a reducing agent prior to the coupling, the reducing agent is preferably DTT. Additionally or alternatively, the formation of free sulfhydryl groups on the protein can also be performed *in situ.* To that end, preferably the coupling is carried out in the presence of a reducing agent. In that case, it is preferred to use a reducing agent that does not contain free sulfhydryl groups itself. Thus, if the coupling is carried out in the presence of a reducing agent, it is preferred that the reducing agent is TCEP.

The skilled person is aware of suitable conditions to carry out the method of synthesizing a conjugate of the disclosure.

Prefeably, the coupling is carried out at a temperature of from 0°C to 40°C, more preferably of from 1°C to 30°C, more preferably still of from 2°C to 20°C, even more preferably of from 4°C to 10°C, and most preferably at about 4°C.

Preferably, the coupling is carried out in an aqueous solution, preferably the aqueous solution is an aqueous buffer solution.

Preferably the coupling is carried out at a pH of from 6.0 to 8.5, preferably of from 6.2 to 8.0, more preferably of from 6.4 to 7.8, even more preferably of from 6.5 to 7.4, more preferably still of from 6.6 to 7.0, and most preferably at a pH of about 6.8.

The present disclosure is herein described with respect to particular embodiments, but the disclosure is not limited thereto but only by the claims. Where an indefinite or definite article is used when referring to a singular noun e.g. "a" or "an", "the", this includes a plural of that noun unless something else is specifically stated.

The verb "to comprise", and its conjugations, as used in this description and in the claims is used in its non-limiting sense to mean that items following the word are included, but items not specifically mentioned are not excluded.

In addition, reference to an element by the indefinite article "a" or "an" does not exclude the possibility that more than one of the element is present, unless the context clearly requires that there is one and only one of the elements. The indefinite article "a" or "an" thus usually means "at least one".

Thus, the scope of the expression "a device comprising means A and B" should not be limited to devices consisting only of components A and B. It means that with respect to the present disclosure, the only relevant components of the device are A and B.

The compounds herein may occur in different tautomeric forms. The compounds according to the disclosure are meant to include all tautomeric forms, unless stated otherwise. When the structure of a compound is depicted as a specific tautomer, it is to be understood that the disclosure of the present application is not limited to that specific tautomer, unless stated otherwise.

The compounds herein may occur in different enantiomeric forms. The compounds according to the disclosure are meant to include all enantiomeric forms, unless stated otherwise. When the structure of a compound is depicted as a specific enantiomer, it is to be understood that the disclosure of the present application is not limited to that specific enantiomer, unless stated otherwise.

Unless stated otherwise, the compounds of the disclosure and/or groups thereof may be protonated or deprotonated. It will be understood that it is possible that a compound may bear multiple charges which may be of opposite sign. For example, in a compound containing an amine and a carboxylic acid, the amine may be protonated while simultaneously the carboxylic acid is deprotonated.

Unless stated otherwise, if in this disclosure reference is made to a molecular structure, such as "compound", "diene", "tetrazine", and the like, it will be understood that such a molecular structure may also be in its salt, hydrate, and/or solvate form.

In several formulae, groups or substituents are indicated with reference to letters such as "A", "B", "X", "Y", and various (numbered) "R" groups. In addition, the number of repeating units may be referred to with a letter, e.g. n in -(CH₂)ₙ-. The definitions of these letters are to be read with reference to each formula, i.e. in different formulae these letters, each independently, can have different meanings unless indicated otherwise.

Herein, reference is made to "alkyl", and the like. The number of carbon atoms that these groups have, excluding the carbon atoms comprised in any optional substituents according to Radical Group 1, can be indicated by a designation preceding such terms (e.g. "C₁-C₈ alkyl" means that said alkyl may have from 1 to 8 carbon atoms). For the avoidance of doubt, a butyl group substituted with a -OCH₃ group is designated as a C₄ alkyl, because the carbon atom in the substituent is not included in the carbon count.

A cycloalkyl group is a cyclic alkyl group. Unsubstituted cycloalkyl groups comprise at least three carbon atoms and have the general formula CₙH₂ₙ₋₁. Optionally, the cycloalkyl groups are substituted by one or more substituents further specified in this document. Examples of cycloalkyl groups include cyclopropyl, cyclobutyl, cyclopentyl and cyclohexyl.

An alkenyl group comprises one or more carbon-carbon double bonds, and may be linear or branched. Unsubstituted alkenyl groups comprising one C-C double bond have the general formula CₙH₂ₙ₋₁. Unsubstituted alkenyl groups comprising two C-C double bonds have the general formula CₙH₂ₙ₋₃. An alkenyl group may comprise a terminal carbon-carbon double bond and/or an internal carbon-carbon double bond. A terminal alkenyl group is an alkenyl group wherein a carbon-carbon double bond is located at a terminal position of a carbon chain. An alkenyl group may also comprise two or more carbon-carbon double bonds. Examples of an alkenyl group include ethenyl, propenyl, isopropenyl, t-butenyl, 1,3-butadienyl, 1,3-pentadienyl, etc. Unless stated otherwise, an alkenyl group may optionally be substituted with one or more, independently selected, substituents according to Radical Group 1.

A cycloalkenyl group is a cyclic alkenyl group. An unsubstituted cycloalkenyl group comprising one double bond has the general formula CₙH₂ₙ₋₃. Optionally, a cycloalkenyl group is substituted by one or more substituents further specified in this document. An example of a cycloalkenyl group is cyclopentenyl.

An alkynyl group comprises one or more carbon-carbon triple bonds, and may be linear or branched. Unsubstituted alkynyl groups comprising one C-C triple bond have the general formula CₙH₂ₙ₋₃. An alkynyl group may comprise a terminal carbon-carbon triple bond and/or an internal carbon-carbon triple bond. A terminal alkynyl group is an alkynyl group wherein a carbon-carbon triple bond is located at a terminal position of a carbon chain. An alkynyl group may also comprise two or more carbon-carbon triple bonds. Unless stated otherwise, an alkynyl group may optionally be substituted with one or more, independently selected, substituents according to Radical Group 1. Examples of an alkynyl group include ethynyl, propynyl, isopropynyl, t-butynyl, etc.

A cycloalkynyl group is a cyclic alkynyl group. An unsubstituted cycloalkynyl group comprising one triple bond has the general formula CₙH₂ₙ₋₅. Optionally, a cycloalkynyl group is substituted by one or more substituents further specified in this document. An example of a cycloalkynyl group is cyclooctynyl.

An aryl group refers to an aromatic hydrocarbon ring system that comprises six to twenty-four carbon atoms, more preferably six to twelve carbon atoms, and may include monocyclic and polycyclic structures. When the aryl group is a polycyclic structure, it is preferably a bicyclic structure. Optionally, the aryl group may be substituted by one or more substituents further specified in this document. Examples of aryl groups are phenyl and naphthyl. Preferably, an aryl group is phenyl.

Arylalkyl groups and alkylaryl groups comprise at least seven carbon atoms and may include monocyclic and bicyclic structures. Optionally, the arylalkyl groups and alkylaryl may be substituted by one or more substituents further specified in this document. An arylalkyl group is for example benzyl. An alkylaryl group is for example 4-tert-butylphenyl.

Preferably, heteroaryl groups comprise five to sixteen carbon atoms and contain between one to five heteroatoms. Heteroaryl groups comprise at least two carbon atoms (i.e. at least C₂) and one or more heteroatoms N, O, P or S. A heteroaryl group may have a monocyclic or a bicyclic structure. Optionally, the heteroaryl group may be substituted by one or more substituents further specified in this document. Examples of suitable heteroaryl groups include pyridinyl, quinolinyl, pyrimidinyl, pyrazinyl, pyrazolyl, imidazolyl, thiazolyl, pyrrolyl, furanyl, triazolyl, benzofuranyl, indolyl, purinyl, benzoxazolyl, thienyl, phospholyl and oxazolyl.

Heteroarylalkyl groups and alkylheteroaryl groups comprise at least three carbon atoms (i.e. at least C₃) and may include monocyclic and bicyclic structures. Optionally, the heteroaryl groups may be substituted by one or more substituents further specified in this document.

Where an aryl group is denoted as a (hetero)aryl group, the notation is meant to include an aryl group and a heteroaryl group. Similarly, an alkyl(hetero)aryl group is meant to include an alkylaryl group and an alkylheteroaryl group, and (hetero)arylalkyl is meant to include an arylalkyl group and a heteroarylalkyl group. A C₂-C₂₄ (hetero)aryl group is thus to be interpreted as including a C₂-C₂₄ heteroaryl group and a C₆-C₂₄ aryl group. Similarly, a C₃-C₂₄ alkyl(hetero)aryl group is meant to include a C₇-C₂₄ alkylaryl group and a C₃-C₂₄ alkylheteroaryl group, and a C₃-C₂₄ (hetero)arylalkyl is meant to include a C₇-C₂₄ arylalkyl group and a C₃-C₂₄ heteroarylalkyl group.

In general, when (hetero) is placed before a group, it refers to both the variant of the group without the prefix hetero- as well as the group with the prefix hetero-. Herein, the prefix hetero- denotes that the group contains one or more heteroatoms selected from the group consisting of O, N, S, P, and Si. Preferably, the one or more heteroatoms is selected from the group consisting of O, N, S, and P. It will be understood that for any compound containing a heteroatom, the N, S, and P atoms are optionally oxidized and the N atoms are optionally quaternized. Preferably, up to two heteroatoms are consecutive, such as in for example -CH₂-NH-OCH₃ and -CH₂-O-Si(CH₃)₃. More preferably, however, the heteroatoms are not directly bound to one another.

Examples of heteroalkyls include -CH₂CH₂-O-CH₃, -CH₂CH₂-NH-CH₃, -CH₂CH₂-S(O)-CH₃, -CH₌CH-O-CH₃, CH₂CH₂-NH₂, CH₂CH₂-SH, -CH₂CH₂-OH, -CH₂CH₂-COOH, - CH₂C(O)H, -C(O)HCH₃, and -Si(CH₃)₃. Preferably, a C₁-C₄ heteroalkyl contains at most 2 heteroatoms.

Herein, it will be understood that when the prefix hetero- is used for combinations of groups, the prefix hetero- only refers to the one group before it is directly placed. For example, heteroarylalkyl denotes the combination of a heteroaryl group and an alkyl group, not the combination of a heteroaryl and a heteroalkyl group.

Herein, the prefix cyclo- denotes that groups are cyclic. It will be understood that when the prefix cyclo- is used for combinations of groups, the prefix cyclo- only refers to the one group before it is directly placed. For example, cycloalkylalkenylene denotes the combination of a cycloalkylene group (see the definition of the suffix -ene below) and an alkenylene group, not the combination of a cycloalkylene and a cycloalkenylene group. In general, when (cyclo) is placed before a group, it refers to both the variant of the group without the prefix cyclo- as well as the group with the prefix cyclo-.

Herein, the suffix -ene denotes divalent groups, i.e. that the group is linked to at least two other moieties. An example of an alkylene is propylene (-CH₂-CH₂-CH₂-), which is linked to another moiety at both termini. It is understood that if a group with the suffix -ene is substituted at one position with -H, then this group is identical to a group without the suffix. For example, an alkylene attached to an -H is identical to an alkyl group. I.e. propylene, - CH₂-CH₂-CH₂-, attached to an -H at one terminus, -CH₂-CH₂-CH₂-H, is logically identical to propyl, -CH₂-CH₂-CH₃.

Herein, when combinations of groups are listed with the suffix -ene, it refers to a divalent group, i.e. that the group is linked to at least two other moieties, wherein each group of the combination contains one linkage to one of these two moieties. As such, for example alkylarylene is understood as a combination of an arylene group and an alkylene group. An example of an alkylarylene group is -phenyl-CH₂-, and an example of an arylalkylene group is -CH₂-phenyl-.

Herein, the suffix -triyl denotes trivalent groups, i.e. that the group is linked to at least three other moieties. An example of an arenetriyl is depicted below: 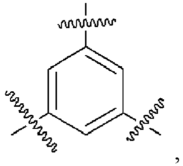 wherein the wiggly lines denote bonds to different groups of the main compound.

It is understood that if a group with the suffix -triyl is substituted at one position with - H, then this group is identical to a divalent group with the suffix -ene. For example, an arenetriyl substituted with -H is identical to an arylene group. Similarly, it is understood that if a group with the suffix -triyl is substituted at two positions with -H, then this group is identical to a monovalent group. For example, an arenetriyl substituted with two -H is identical to an aryl group.

Unless indicated otherwise, a hetero group may contain a heteroatom at non-terminal positions or at one or more terminal positions. In this case, "terminal" refers to the terminal position within the group, and not necessarily to the terminal position of the entire compound. For example, C₂ heteroalkylene may refer to -NH-CH₂-CH₂-, -CH₂-NH-CH₂-, and -CH₂-CH₂-NH-. For example, C₂ heteroalkyl may refer to -NH-CH₂-CH₃, -CH₂-NH-CH₃, and -CH₂-CH₂-NH₂.

Herein, it is understood that cyclic compounds (i.e. aryl, cycloalkyl, cycloalkenyl, etc.) are understood to be monocyclic, polycyclic or branched. It is understood that the number of carbon atoms for cyclic compounds not only refers to the number of carbon atoms in one ring, but that the carbon atoms may be comprised in multiple rings. These rings may be fused to the main ring or substituted onto the main ring. For example, C₁₀ aryl optionally containing heteroatoms may refer to *inter alia* a naphthyl group (fused rings) or to *e.g.* a bipyridyl group (substituted rings, both containing an N atom).

Unless stated otherwise, any group disclosed herein that is not cyclic is understood to be linear or branched. In particular, (hetero)alkyl groups, (hetero)alkenyl groups, (hetero)alkynyl groups, (hetero)alkylene groups, (hetero)alkenylene groups, (hetero)alkynylene groups, and the like are linear or branched, unless stated otherwise.

As used herein, unless stated otherwise all of the following groups: (hetero)alkyl, (hetero)alkenyl, (hetero)alkynyl, (hetero)cycloalkyl, (hetero)cycloalkenyl, (hetero)cycloalkynyl, (hetero)aryl, (hetero)alkylene, (hetero)alkenylene, (hetero)alkynylene, (hetero)cycloalkylene, (hetero)cycloalkenylene, (hetero)cycloalkynylene, (hetero)arylene, (hetero)alkanetriyl, (hetero)cycloalkanetriyl, arenetriyl, heteroarenetriyl, combinations thereof, and the like, can be substituted or unsubstituted; preferably these groups are unsubstituted. If said groups are substituted, said groups preferably contain up to 4, more preferably up to 3, more preferably still up to 2, and most preferably 1 substituent according to Radical Group 1 as defined herein.

The term "protein" is herein used in its normal scientific meaning. Herein, polypeptides comprising about 10 or more amino acids are considered proteins. A protein may comprise natural, but also unnatural amino acids.

The term "salt thereof" means a compound formed when an acidic proton, typically a proton of an acid, is replaced by a cation, such as a metal cation or an organic cation and the like. The term "salt thereof" also means a compound formed when an amine is protonated. Where applicable, the salt is a pharmaceutically acceptable salt, although this is not required for salts that are not intended for administration to a patient. For example, in a salt of a compound the compound may be protonated by an inorganic or organic acid to form a cation, with the conjugate base of the inorganic or organic acid as the anionic component of the salt.

The term "pharmaceutically accepted salt" means a salt that is acceptable for administration to a patient, such as a mammal (salts with counter-ions having acceptable mammalian safety for a given dosage regime). Such salts may be derived from pharmaceutically acceptable inorganic or organic bases and from pharmaceutically acceptable inorganic or organic acids.

"Pharmaceutically acceptable salt" refers to pharmaceutically acceptable salts of a compound, which salts are derived from a variety of organic and inorganic counter ions known in the art and include, for example, sodium, potassium, calcium, magnesium, ammonium, tetraalkylammonium, etc., and when the molecule contains a basic functionality, salts of organic or inorganic acids, such as hydrochloride, hydrobromide, formate, tartrate, besylate, mesylate, acetate, maleate, oxalate, etc.

As used herein, the term "solvate" refers to a compound that apart from a main molecule *(e.g.* a compound of the disclosure, a diene, and the like) further includes a stoichiometric or non-stoichiometric amount of solvent bound to said main molecule by noncovalent intermolecular forces. **In** particular, the term "solvate" may refer to a crystalline compound, the crystal lattice structure of which contains one or more molecules of the solvent.

As used herein, the term "hydrate" refers to a compound that apart from a main molecule *(e.g.* a compound of the disclosure, a diene, and the like) further includes a stoichiometric or non-stoichiometric amount of water bound to said main molecule by noncovalent intermolecular forces. **In** particular, the term "hydrate" may refer to a crystalline compound, the crystal lattice structure of which contains one or more molecules of water.

The logarithm of the partition-coefficient, i.e. Log **P,** is herein used as a measure of the hydrophobicity of a compound. Typically, the Log P is defined as $log \left(\frac{{\left[Solute\right]}_{octanol}^{un - ionized}}{{\left[Solute\right]}_{water}^{un - ionized}}\right)$ The skilled person is aware of methods to determine the partition-coefficient of compounds without undue experimentation. Alternatively, the skilled person knows that software is available to reliably estimate the Log P value, for example as a function within ChemDraw^{®} software or online available tools.

The unified atomic mass unit or Dalton is herein abbreviated to Da. The skilled person is aware that Dalton is a regular unit for molecular weight and that 1 Da is equivalent to 1 g/mol (grams per mole).

It will be understood that herein, the terms "moiety" and "group" are used interchangeably when referring to a part of a molecule.

It will be understood that when a heteroatom is denoted as -X(R')₂-, wherein X is the heteroatom and R' is a certain moiety, then this denotes that two moieties R' are attached to the heteroatom.

It will be understood that when a group is denoted as, for example, -((R₅₁)₂-R₅₂)₂- or a similar notation, in which R₅₁ and R₅₂ are certain moieties, then this denotes that first, it should be written as -R₅₁-R₅₁-R₅₂-R₅₁-R₅₁-R₅₂- before the individual R₅₁ and R₅₂ moieties are selected, rather than first selecting moieties R₅₁ and R₅₂ and then writing out the formula.

As used herein, "activated carboxylic acid" and "active ester" may be used interchangeably. As the skilled person is aware, an "activated carboxylic acid" or an "active ester" is a derivative of a carboxylic acid (-C(O)OH) of which the -OH moiety has been exchanged for a better leaving group. Preferred activated carboxylic acids or active esters are selected from the group consisting of -C(O)O-N-succinimidyl, -C(O)O-pentafluorophenyl, - C(O)O-tetrafluorophenyl, -C(O)O-4-nitrophenyl, and -C(O)Cl. More preferably, the activated carboxylic acid or active ester is -C(O)O-N-succinimidyl, or -C(O)O-pentafluorophenyl.

As used herein, a "drug" refers to a pharmaceutical agent. As such, "drug", "pharmaceutical agent", "therapeutic agent", and "medicine" can typically be used interchangeably. Drugs in relation to the disclosure are monomethyl auristatin E (MMAE), exatecan, and exatecan derivatives. As used herein, exatecan and exatecan derivatives have the following structure: 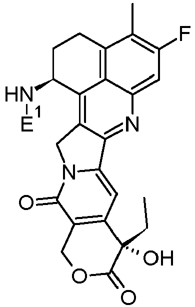 wherein E¹ is -H or -CH₃. It will be understood that when E¹ is -H, said structure is exatecan. Preferably, the exatecan or exatecan derivative is linked to the remainder of R₄₈ via the nitrogen atom to which E¹ is attached. Most preferably, E¹ is -H. Most preferably, the drug is monomethyl auristatin E (MMAE).

### Radical groups (RG)

### Radical Group 1: terminal groups

For Radical Group 1 (RG1), the radical is selected from the group consisting of -H, - Cl, -F, -Br, -I, -OH, -NH₂, -COOH, -CONH₂, -CN, -N₃, -NCS, -SCN, -SO₃H, -PO₃H, -PO₄H₂, -N0₂, -CF₃, -CF₂H, -CFH₂, =O, =NH, -SH, -SO₂H, (hetero)alkyl, (hetero)alkenyl, (hetero)alkynyl, (hetero)cycloalkyl, (hetero)cycloalkenyl, (hetero)cycloalkynyl, (hetero)aryl, and combinations thereof.

For RG1, "combinations thereof" in particular refers to (hetero)alkylcycloalkyl, (hetero)alkylcycloalkenyl, (hetero)alkylcycloalkynyl, (hetero)cycloalkylalkyl, (hetero)cycloalkenylalkyl, (hetero)cycloalkynylalkyl, (hetero)alkenylcycloalkyl, (hetero)alkenylcycloalkenyl, (hetero)alkenylcycloalkynyl, (hetero)cycloalkylalkenyl, (hetero)cycloalkenylalkenyl, (hetero)cycloalkynylalkenyl, (hetero)alkynylcycloalkyl, (hetero)alkynylcycloalkenyl, (hetero)alkynylcycloalkynyl, (hetero)cycloalkylalkynyl, (hetero)cycloalkenylalkynyl, (hetero)cycloalkynylalkynyl, (hetero)arylalkyl, (hetero)arylalkenyl, (hetero)arylalkynyl, alkyl(hetero)aryl, alkenyl(hetero)aryl, alkynyl(hetero)aryl, cycloalkyl(hetero)aryl, cycloalkenyl(hetero)aryl, cycloalkynyl(hetero)aryl, (hetero)arylcycloalkyl, (hetero)arylcycloalkenyl, and (hetero)arylcycloalkynyl. In addition, "combinations thereof" in relation to RG1 also refers to *e.g.* an alkyl group substituted with one or more -Cl and/or -OH groups. As such, RG1 also comprises radicals such as -NH-CH₂-COOH (a glycine residue), which is a combination of a heteroalkyl and -COOH.

Preferably, for RG1 the radical is selected from the group RG1a consisting of -H, -Cl, -F, -Br, -I, -OH, -NH₂, -COOH, -CONH₂, -SO₃H, -PO₃H, -PO₄H₂, -NO₂, -CF₃, =O, =NH, -SH, -(S^{P})ᵢ-C^{B}, C₁-C₂₄ (hetero)alkyl, C₂-C₂₄ (hetero)alkenyl, C₂-C₂₄ (hetero)alkynyl, C₃-C₂₄ cycloalkyl, C₂-C₂₄ heterocycloalkyl, C₅-C₂₄ cycloalkenyl, C₃-C₂₄ heterocycloalkenyl, C₇-C₂₄ cycloalkynyl, C₅-C₂₄ (hetero)cycloalkynyl, C₆-C₂₄ aryl, C₂-C₂₄ heteroaryl, and combinations thereof.

More preferably, for RG1 the radical is selected from the group RG1b consisting of - H, -Cl, -F, -Br, -I, -OH, -NH₂, -COOH, -CONH₂, -SO₃H, -PO₃H, -PO₄H₂, -NO₂, -CF₃, =O, =NH, -SH, -(S^{P})ᵢ-C^{B}, C₁-C₁₂ (hetero)alkyl, C₂-C₁₂ (hetero)alkenyl, C₂-C₁₂ (hetero)alkynyl, C₃-C₁₂ cycloalkyl, C₂-C₁₂ heterocycloalkyl, C₅-C₁₂ cycloalkenyl, C₃-C₁₂ heterocycloalkenyl, C₇-C₁₂ cycloalkynyl, C₅-C₁₂ (hetero)cycloalkynyl, C₆-C₁₂ aryl, C₂-C₁₂ heteroaryl, and combinations thereof.

Even more preferably, for RG1 the radical is selected from the group RG1c consisting of -H, -Cl, -F, -Br, -I, -OH, -NH₂, -COOH, -CONH₂, -SO₃H, -PO₃H, -PO₄H₂, -N0₂, -CF₃, =O, =NH, -SH, -(S^{P})ᵢ-C^{B}, C₁-C₈ (hetero)alkyl, C₂-C₈ (hetero)alkenyl, C₂-C₈ (hetero)alkynyl, C₃-C₈ cycloalkyl, C₂-C₈ heterocycloalkyl, C₅-C₈ cycloalkenyl, C₃-C₈ heterocycloalkenyl, C₇-C₈ cycloalkynyl, C₅-C₈ (hetero)cycloalkynyl, C₆-C₈ aryl, C₂-C₈ heteroaryl, and combinations thereof.

More preferably still, for RG1 the radical is selected from the group RG1d consisting of -H, -Cl, -F, -Br, -I, -OH, -NH₂, -COOH, -CONH₂, -SO₃H, -PO₃H, -PO₄H₂, -N0₂, -CF₃, =O, =NH, -SH, -(S^{P})ᵢ-C^{B}, C₁-C₆ (hetero)alkyl, C₂-C₆ (hetero)alkenyl, C₂-C₆ (hetero)alkynyl, C₃-C₆ cycloalkyl, C₂-C₆ heterocycloalkyl, C₅-C₇ cycloalkenyl, C₃-C₅ heterocycloalkenyl, C₈ cycloalkynyl, C₆-C₇ (hetero)cycloalkynyl, phenyl, C₃-C₅ heteroaryl, and combinations thereof.

Most preferably, for RG1 the radical is selected from the group RG1e consisting of -H, -Cl, -F, -Br, -I, -OH, -NH₂, -COOH, -CONH₂, -SO₃H, -PO₃H, -PO₄H₂, -NO₂, -CF₃, =O, =NH, -SH, -(S^{P})ᵢ-C^{B}, C₁-C₃ (hetero)alkyl, C₃-C₆ cycloalkyl, C₂-C₅ heterocycloalkyl, phenyl, C₄-C₅ heteroaryl, and combinations thereof.

In some embodiments, for RG1 the radical is a conjugation moiety, which is a chemical group that can be used for binding, conjugation or coupling of a Construct, such as Construct-B, or a Spacer, or another molecule or construct of interest. The person skilled in the art is aware of the myriad of strategies that are available for the chemoselective or -unselective or enzymatic coupling or conjugation of one molecule or construct to another.

In some embodiments, RG1 is a moiety that allows conjugation to a protein comprising natural and/or non-natural amino acids. Moieties suitable for conjugation are known to the skilled person. Conjugation strategies are for example found in [O. Boutureira, G.J.L. Bernardes, Chem. Rev., 2015, 115, 2174-2195].

If RG1 is a conjugation moiety, it is preferably selected from the group RG1f consisting of N-maleimidyl, halogenated N-alkylamido, sulfonyloxy N-alkylamido, vinyl sulfone, (activated) carboxylic acids, active ester, benzenesulfonyl halides, ester, carbonate, sulfonyl halide, thiol or derivatives thereof, C₂₋₆ alkenyl, C₂₋₆ alkynyl, C₇₋₁₈ cycloalkynyl, C₅₋₁₈ heterocycloalkynyl, bicyclo[6.1.0]non-4-yn-9-yl], C₃₋₁₂ cycloalkenyl, azido, phosphine, nitrile oxide, nitrone, nitrile imine, isonitrile, diazo, ketone, (O-alkyl)hydroxylamino, hydrazine, halogenated N-maleimidyl, aryloxymaleimides, dithiophenolmaleimides, bromo- and dibromopyridazinediones, 2,5-dibromohexanediamide, alkynone, 3-arylpropiolonitrile, 1,1-bis(sulfonylmethyl)-methylcarbonyl or elimination derivatives thereof, carbonyl halide, allenamide, 1,2-quinone, isothiocyanate, isocyanate, aldehyde, triazine, squaric acids, 2-imino-2-methoxyethyl, (oxa)norbornene, (oxa)norbornadiene, (imino)sydnones, methylsulfonyl phenyloxadiazole, aminooxy, 2-amino benzamidoxime, ethynylphosphonamidates, reactive in the Pictet-Spengler ligation and hydrazine-Pictet-Spengler (HIPS) ligation, DNA intercalators, tetrazine, trans-cyclooctene, and photocrosslinkers. More preferably, RG1f is N-maleimidyl.

In other embodiments RG1f is selected from the group consisting of hydroxyl, amine, halogens, vinyl pyridine, disulfide, pyridyl disulfide, sulfonyloxy, mercaptoacetamide, anhydride, sulfonylated hydroxyacetamido, sulfonyl chlorides, thiosemicarbazone, hydrazine carboxylate, and arylhydrazide. In yet other embodiments RG1f is a group that can be connected to another group by means of an enzyme, for example sortase or Tubulin tyrosine ligase.

### Examples

### Example 1: Synthesis

### Example 1a: compounds 1 and 2

Intermediate compound 3 was obtained in three steps with an overall yield of 29% using commercially available starting materials:

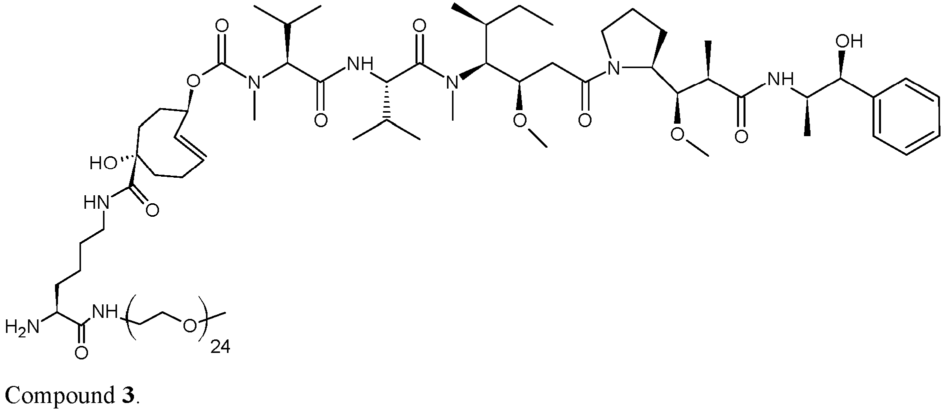

Compounds **4** and **5** were synthesized by coupling compound **3** to maleimide-PEG4-COOH or maleimide-C5-COOH, respectively, using conventional coupling reagents. This afforded the desired products, viz. compounds **4** and **5,** in high yield.

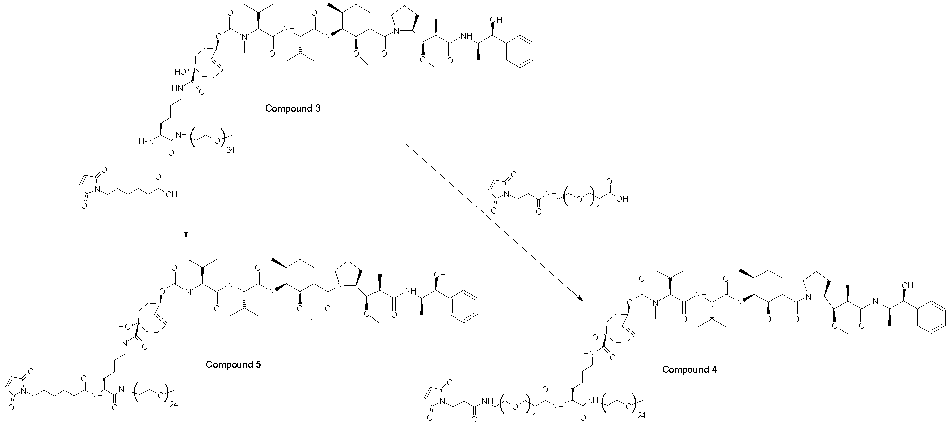

Compound **4** or **5,** respectively, was conjugated to diabody AVP0458 to afford compound **1** or **2** following the optimized procedure by Rossin et al., Nature Communications (2018)9:1484.

Briefly, 10 mg AVP0458 was reacted with 6 mM DTT for 3 h at room temperature on a roller bench, followed by purification via PD-10 pre-equilibrated with 0.1 M phosphate buffer pH 6.8, containing 2 mM EDTA (PB-EDTA buffer). The purified diabody solution was then split in two aliquots which were then added with 30 eq of compound **4** or compound **5,** respectively, dissolved in dry DMSO at 10 mM concentration. The two reaction mixtures were incubated for 1h at room temperature on a roller bench followed by overnight incubation at +4°C. The two resulting conjugates ADCs (compound **1,** conjugate of AVP0458 and compound **4;** and compound **2,** conjugate of AVP0458 and compound **5)** were then purified from the crude mixtures by SEC (Superdex75 10/300 column eluted with PBS at 0.8 mL/min) followed by concentration via Amicon Ultra-4 (30 kDa MW cut-off). UV measurements on the final solutions showed 75-80% recovery of diabody. SDS-PAGE analysis of the two ADC solutions showed the presence of one species with the expected increase in MW with respect to that of the monomer in AVP0458. Further analysis using mass spectrometry showed a complete reaction between the four cysteine residues in AVP0458 with the respective TCOs, confirming the production of two conjugates with a DAR of 4.

Compound **1** has the following structure:

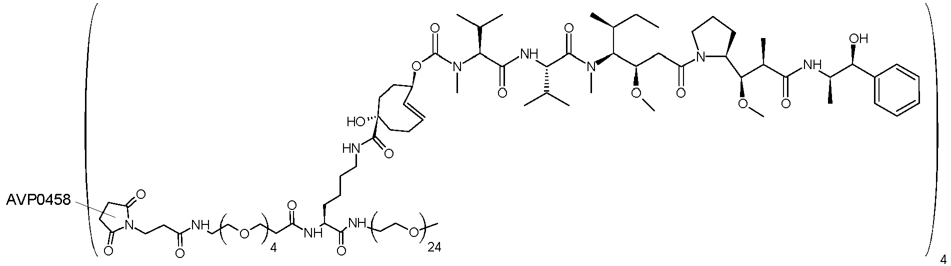

Compound 2 has the following structure:

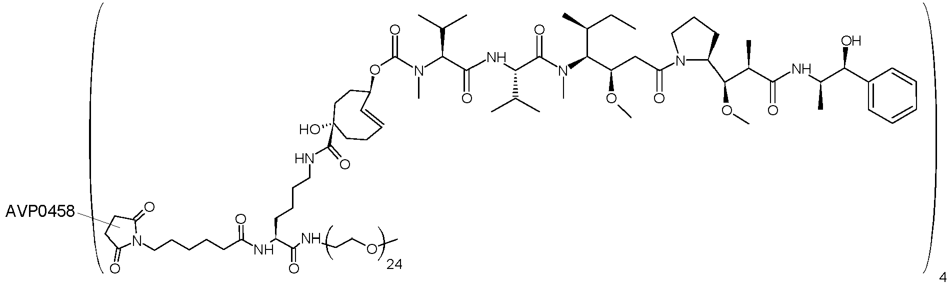

### Example 1b: reference compounds 14a and 14b and claimed compounds 15a and 15b

Reference compounds **14a** and **14b** were synthesized by conjugating compound **11a** or **11b**, respectively, to the diabody AVP0458. Likewise, claimed compounds **15a** and **15b** were synthesized by conjugating compound **13a** or **13b,** respectively, to the diabody AVP0458. Below, first the synthesis of **11a** and **11b** is described, and then the synthesis of **13a** and **13b.** Thereafter, the conjugation is described of **11a, 11b, 13a,** or **13b** to AVP0458 to yield **14a, 14b, 15a,** or **15b.**

### Example 1b-i: synthesis of compounds 11a and 11b

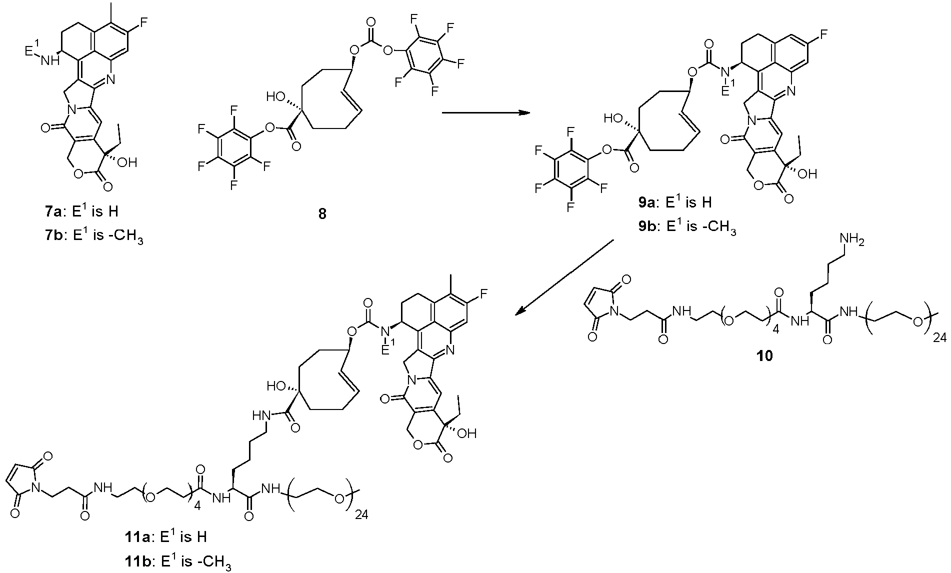

Compound **11a** was prepared in several steps *in situ.* To a suspension of exatecan mesylate (7) (287 mg, 0.54 mmol, MedChemExpress) in 6 mL of anhydrous dimethylformamide (DMF) in a glass vial was added compound **8** (506 mg, 0.90 mmol) and diethylamine (DIEA; 313 µL, 1.80 mmol). The mixture was stirred at room temperature in the dark for 2 h, at which point LC-MS analysis indicated complete consumption **of 7a and** formation of intermediate **9a.** The excess of compound **8** was quenched by addition of N-isopropylmethylamine (73 mg, 1.0 mmol) and stirring at room temperature in the dark for 2 h. To this reaction mixture was added a solution of compound **10** (trifluoroacetic acid salt, 1555 mg, 0.90 mmol) and DIEA (312 µL, 1.80 mmol) in 4 mL of anhydrous DMF. The reaction mixture was stirred at room temperature in the dark for 2 h. The mixture was purified by preparative RP-HPLC (HPLC conditions: solvent A (0.05% TFA in water), solvent B (acetonitrile), 10 to 65% of B over 30 min at a rate of 50 mL/min, elution time 24 min). The collected fractions were analyzed by HPLC/LC-MS. The pure fractions were lyophilized in the dark to give compound **11a.** (234 mg, 19 % yield from 7). LCMS m/z 1122.7 ((M+2H)/2).

Compound **11b** is prepared in several steps *in situ.* To N-methyl exatecan **(7b,** 1 eq) in 6 mL of anhydrous dimethylformamide (DMF) in a glass vial is added compound **8** (1 eq) and diethylamine (4 eq). The mixture is stirred at room temperature in the dark for 11 days. To this reaction mixture is added a solution of compound **10** (trifluoroacetic acid salt, 1 eq) and DIEA (4 eq) in 4 mL of anhydrous DMF. The reaction mixture is stirred at room temperature in the dark for 2 h. The mixture is purified by preparative RP-HPLC (HPLC conditions: solvent A (0.05% TFA in water), solvent B (acetonitrile), 10 to 65% of B over 30 min at a rate of 50 mL/min. The collected fractions are analyzed by HPLC/LC-MS and pure fractions are lyophilized to give compound 11b.

### Example 1b-ii: synthesis of compounds 13a and 13b

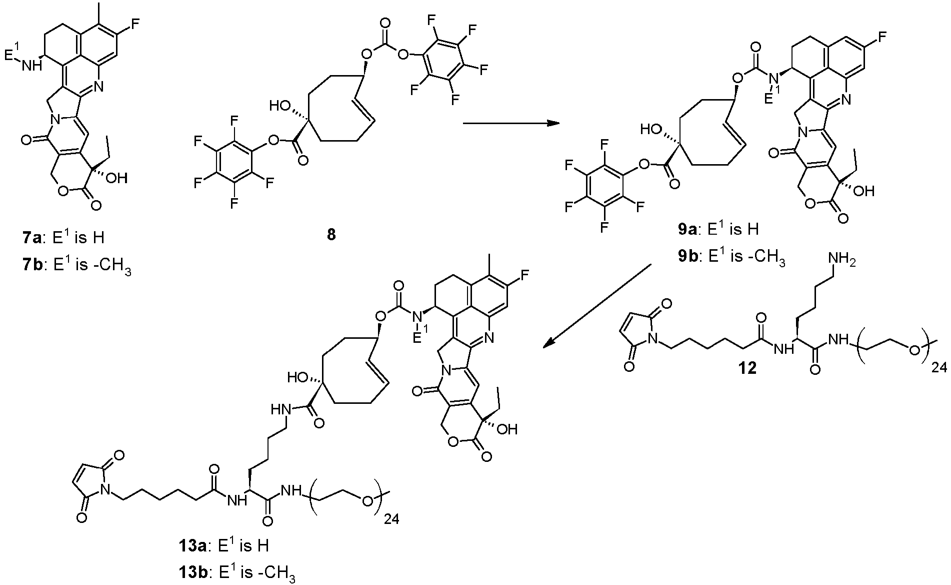

Compound **13a** was prepared in several steps in situ. To a suspension of exatecan mesylate **(7a)** (287 mg, 0.54 mmol, MedChemExpress) in 6 mL of anhydrous DMF in a glass vial was added compound **8** (506 mg, 0.90 mmol) and DIEA (313 µL, 1.80 mmol). The mixture was stirred at room temperature in the dark for 2 h, at which point LC-MS analysis indicated complete consumption of **7a** and formation of intermediate **9a.** The excess of compound **8** was quenched by addition of N-isopropylmethylamine (73 mg, 1.0 mmol) and stirring at room temperature in the dark for 2 h. To this reaction mixture was added a solution of compound **12** (HCl salt, 1300 mg, 0.90 mmol, Biomatrik) and DIEA (156 µL, 0.90 mmol) in 4 mL of anhydrous DMF. The reaction mixture was stirred at room temperature in the dark for 2 h. The mixture was purified by preparative RP-HPLC (HPLC conditions: solvent A (0.05% TFA in water), solvent B (acetonitrile), 10 to 65% of B over 30 min at a rate of 50 mL/min, elution time 26 min). The collected fractions were analyzed by HPLC/LC-MS. The pure fractions were lyophilized in the dark to give compound **13a.** (280 mg, 25 % yield from 7). LCMS m/z 1020.3 ((M+2H)/2).

Compound **13b** is prepared in several steps *in situ.* To N-methyl exatecan **(7b,** 1 eq) in 6 mL of anhydrous dimethylformamide (DMF) in a glass vial is added compound **8** (1 eq) and diethylamine (4 eq). The mixture is stirred at room temperature in the dark for 11 days. To this reaction mixture is added a solution of compound **12** (trifluoroacetic acid salt, 1 eq) and diethylamine (4 eq) in 4 mL of anhydrous DMF. The reaction mixture is stirred at room temperature in the dark for 2 h. The mixture is purified by preparative RP-HPLC (HPLC conditions: solvent A (0.05% TFA in water), solvent B (acetonitrile), 10 to 65% of B over 30 min at a rate of 50 mL/min. The collected fractions are analyzed by HPLC/LC-MS and pure fractions are lyophilized to give compound **13b.**

### Example 1b-iii: synthesis of compounds 14a, 14b, 15a, and 15b

Compound **11a, 11b, 13a,** or **13b** was conjugated to diabody AVP0458 to afford compound **14a, 14b, 15a,** or **15b,** respectively, following the optimized procedure by Rossin et al., Nature Communications (2018)9:1484.

Briefly, 2 mg AVP0458 was reacted with 6 mM DTT for 2 h at room temperature on a roller bench, followed by purification via PD-10 pre-equilibrated with 0.1 M phosphate buffer pH 6.8, containing 2 mM ETDA (PB-EDTA buffer). The purified diabody solution was then split in four aliquots which were then added with 24 eq of compound **11a, 11b, 13a,** or **13b,** respectively, dissolved in dry DMSO at 10 mM concentration. The four reaction mixtures were incubated for 1h at room temperature on a roller bench followed by overnight incubation at +4°C. The four resulting conjugates ADCs (compounds **14a, 14b, 15a,** and **15b,** conjugates of AVP0458 and compound **11a, 11b, 13a,** or **13b,** respectively) were then purified from the crude mixtures by SEC (Superdex75 10/300 column eluted with PBS at 0.8 mL/min) followed by concentration via Amicon Ultra-4 (30 kDa MW cut-off). UV measurements on the final solutions showed 60-78% recovery of diabody. SDS-PAGE analysis of the two ADC solutions showed the presence of one species with the expected increase in MW with respect to that of the monomer in AVP0458. Further analysis using mass spectrometry showed a complete reaction between the four cysteine residues in AVP0458 with the respective TCOs, confirming the production of two conjugates with a DAR of 4.

Conjugates **14a, 14b, 15a,** and **15b** have the following structures:

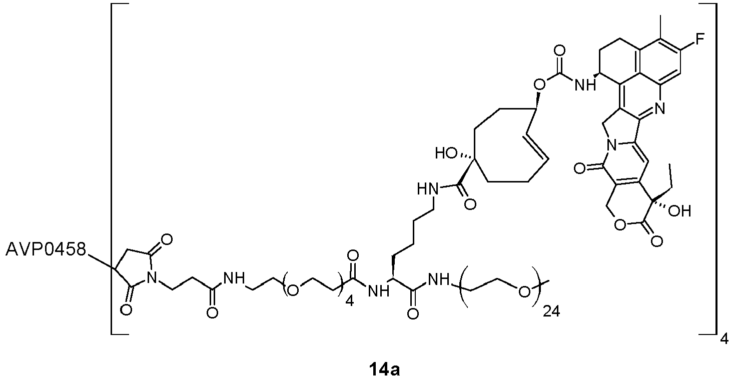

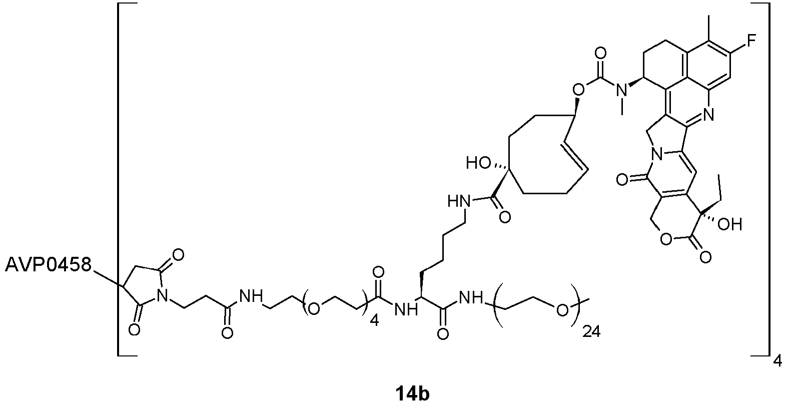

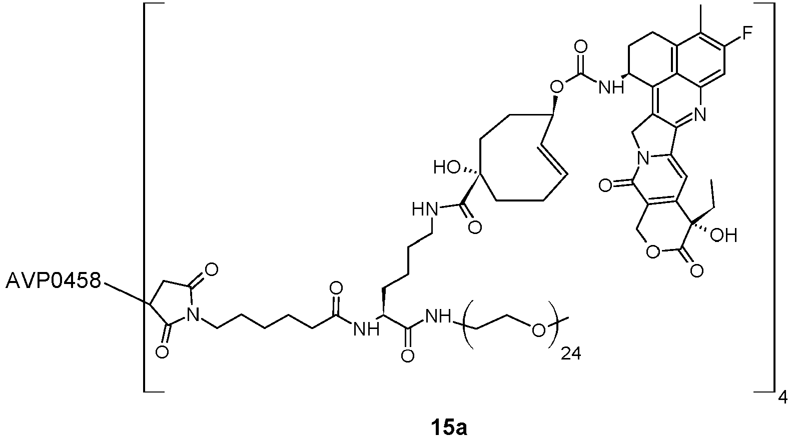

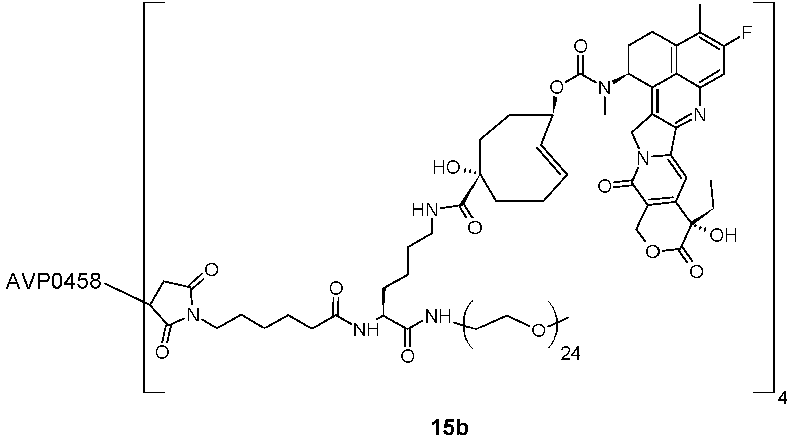

### Example 2: in vivo blood clearance in tumor-free mice

Six groups of tumor-free mice (n = 3 or 4 per group) were injected ¹²⁵I-labeled compound **1, 2, 14a, 15a, 14b,** or **15b in** a dosage of 5 mg/kg (for compounds 1, **2, 14a,** and **15a)** or 1 mg/kg (for compounds **14b** and **15b).** Blood samples (ca 50 µL) were withdrawn from the vena saphena at various times up to 72h post injection.

For experiments using compounds **1, 2, 14a,** or **15a,** after gamma-counting plasma isolated from blood was reacted *ex vivo* with an excess of tetrazine **6** for at least 1h at 37°C.

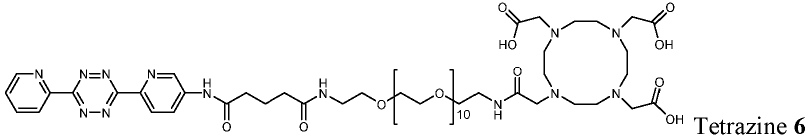

Then, the samples were analyzed by SEC on a Superdex75 10/300 column eluted with PBS at 0.8 mL/min. The eluates were collected in 1 ml fractions which were then measured by gamma-counting using a dual-isotope protocol with crossover correction.

From the above exeperiments, the amount of compound **1, 2, 14a, 15a, 14b,** or **15b** in the blood of the mice 48 hours after injection could be determined, as well as the respective half-lives in blood for said compounds. The results are shown in Table 1.

**Table 1. In vivo blood clearance in tumor-free mice.**

| | **Compound left in blood of mice 48h post-injection** (in %ID/g) | **Half-lives of compounds in blood of mice** (in hours) |
|---|---|---|
| Compound **1** (reference) | 1.14 ± 0.15 | 4.22 |
| Compound **2** | 0.82 ± 0.06 | 4.20 |
| Compound **14a** (reference) | 1.36 ± 0.19 | 5.22 |
| Compound **15a** | 0.92 ± 0.16 | 4.82 |
| Compound **14b** (reference) | 1.02 ± 0.40 | 4.98 |
| Compound **15b** | 0.85 ± 0.09 | 4.53 |

Thus, compound **2** advantageously and surprisingly has a faster clearance rate than compound **1.** Likewise, compounds **15a** and **15b** advantageously and surprisingly have faster clearance rates than compounds **14a** and **14b,** respectively. Furthermore, it was observed that at least compounds **1, 2, 14a,** and **15a** showed high *in vivo* TCO stability.

### Example 3: in vivo tumor and off-target binding of in tumour-bearing mice

Below, the *in vivo* tumor binding and off-target binding in tumor-bearing mice of compounds **1, 2, 14a,** and **15a** is described. The protocol for compounds **1** and **2** are discussed first, and then the protocol for compounds **14a** and **15a.** Thereafter, the results are presented in Table 1.

### Example 3-i: protocol for compounds 1 and 2

Two groups of mice (n=5) bearing LS174T xenografts were injected compound **1** (reference) or compound **2** (2 mg/kg) followed 49h later by ¹¹¹In-labeled tetrazine **6** (10 eq with respect to ADC). Three hours post tetrazine **6** injection, the mice were euthanized and blood, tumors and other tissues were harvested, weighed and counted (together with standards) in a gamma counter with dual isotope protocol with crossover correction. The ¹²⁵I counts were used to calculate the amounts of compounds 1 and 2 in the various tissues (as %ID/g).

### Example 3-ii: protocol for compounds 14a and 15a

Two groups of mice (n=4) bearing LS174T xenografts were injected compound **14a** (reference) or compound **15a** (2 mg/kg) The mice were euthanized 52 h post-ADC injection and blood, tumors and other tissues were harvested, weighed and counted together with standards. The ¹²⁵I counts were used to calculate the amounts of compounds 1 and 2 in the various tissues (as %ID/g).

The results of Example 3 are shown in Tables 2 and 3.

**Table 2. Biodistribution of compounds in tumor-bearing mice.**

| | **Amount of compound (in % ID/g)** | | | |
|---|---|---|---|---|
| | **Compound** 1 (reference) | **Compound 2** (claimed) | **Compound 14a** (reference) | **Compound 15a** (claimed) |
| **Tumor** | 18.42 ± 5.14 | 23.11 ± 6.21^{a} | 44.15 ± 2.02 | 55.34 ± 6.97 |
| **Blood** | 0.77 ± 0.12 | 0.52 ± 0.10 | 0.75 ± 0.14 | 0.87 ± 0.46^{b} |
| **Heart** | 0.22 ± 0.04 | 0.15 ± 0.03 | 0.33 ± 0.06 | 0.33 ± 0.12 |
| **Lung** | 0.59 ± 0.06 | 0.44 ± 0.06 | 0.90 ± 0.17 | 1.03 ± 0.30 |
| **Liver** | 0.43 ± 0.20 | 0.38 ± 0.22 | 1.46 ± 0.73 | 1.69 ± 0.73 |
| **Spleen** | 0.27 ± 0.08 | 0.21 ± 0.07 | 0.71 ± 0.31 | 0.64 ± 0.20 |
| **Kidney** | 0.60 ± 0.08 | 0.57 ± 0.14 | 0.93 ± 0.25 | 0.64 ± 0.20 |
| **Muscle** | 0.08 ± 0.02 | 0.06 ± 0.01 | 0.10 ± 0.02 | 0.11 ± 0.04 |
| **Bone** | 0.11 ± 0.01 | 0.10 ± 0.02 | 0.19 ± 0.04 | 0.14 ± 0.10 |

| | | | | |
|---|---|---|---|---|
| ^{a} n = 4 (one mouse did not develop a tumour). ^{b} the individual values are 0.45, 0.64, 0.87, and 1.51 (possible outlier). Without the possible outlier, the mean value is 0.66 ± 0.21 %ID/g. | | | | |

**Table 3. Biodistribution of compounds in tumor-bearing mice.**

| | **Amount of compound (in % ID/organ)** | | | |
|---|---|---|---|---|
| | **Compound 1** (reference) | **Compound 2** (claimed) | **Compound 14a** (reference) | **Compound 15a** (claimed) |
| **Stomach full** | 0.04 ± 0.01 | 0.03 ± 0.01 | 0.10 ± 0.04 | 0.07 ± 0.01 |
| **Small intestine full** | 0.27 ± 0.03 | 0.20 ± 0.04 | 0.42 ± 0.12 | 0.40 ± 0.14 |
| **Large intestine full** | 0.17 ± 0.03 | 0.16 ± 0.04 | 0.42 ± 0.08 | 0.33 ± 0.14 |
| **Thyroid** | 0.48 ± 0.11 | 0.42 ± 0.07 | 1.12 ± 0.58 | 0.44 ± 0.20 |

From Tables 2 and 3 it is clear that compound **2** shows a higher uptake in tumour and a lower off-target uptake than reference compound **1.** Likewise, compound **15a** shows a higher uptake in tumour and a lower off-target uptake than reference compound **14a.** These results are highly advantageous, since the higher the tumour/off target ratio, the lower the extent of unwanted side-effects are usually observed.

### Example 4: further in vitro and in vivo properties of compound 2

Other properties of compound **2** were tested as well:
*1. In vitro* metabolism studies were carried out using compound **1** or **2** in the presence or absence of acidified human liver S9 fraction for up to 24 hours. From these studies, it was shown that compound **2** has a better metabolism profile than compound **1.**
2. An *in vitro* reaction of compound **2** with a standard tetrazine yielded quantitative MMAE release after 24 hours of incubation.
3. At most 0.8% MMAE release was detected after 6 days of incubating compound **2** in mouse plasma in the absence of a tetrazine or any other trigger.

## Claims

1. A compound according to Formula (2): 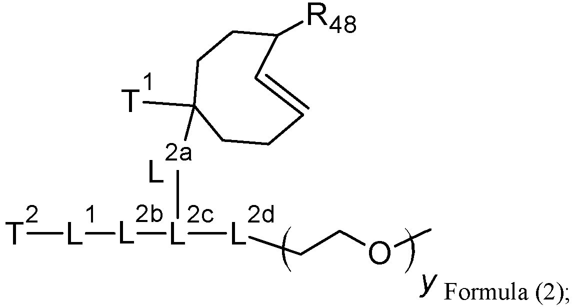 wherein
**L¹ is** a linear C₅-C₆ alkylene;
**T¹ is** -OH;
**R₄₈** is -O-C(O)-C^{A}; wherein C^{A} is a drug, wherein the drug is selected from the group consisting of monomethyl auristatin E, exatecan, and N-methyl-exatecan;
wherein the drug is linked to the moiety -O-C(O)- via a secondary or tertiary nitrogen atom that is part of the drug, forming a carbamate;
**L²a, L^{2b},** and **L^{2d}** are each independently selected from the group consisting of -C(O)NH-, and -NHC(O)-;
**L^{2c}** is C₄-C₆ alkanetriyl;
**T²** is maleimidyl;
y is an integer in a range of from 20 to 30.

2. The compound according to claim 1, wherein L¹ is an unsubstituted, linear C₅ alkylene.

3. The compound according to any one of the preceding claims, wherein T¹ is in an axial position, and wherein R₄₈ is in an axial position.

4. The compound according to any one of the preceding claims, wherein the drug is monomethyl auristatin E (MMAE).

5. The compound according to any one of the preceding claims, wherein L¹ is an unsubstituted, linear C₅ alkylene, y is 24, the drug is monomethyl auristatin E, T¹ is in an axial position, and R₄₈ is in an axial position.

6. The compound according to any one of the preceding claims, wherein said compound is according to Formula (O): 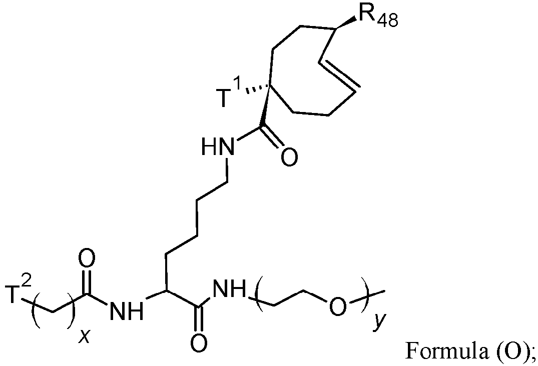 wherein x is 5, y is 24, the drug is monomethyl auristatin E, T¹ is in an axial position, and R₄₈ is in an axial position.

7. The compound according to any one of the preceding claims, wherein said compound is:

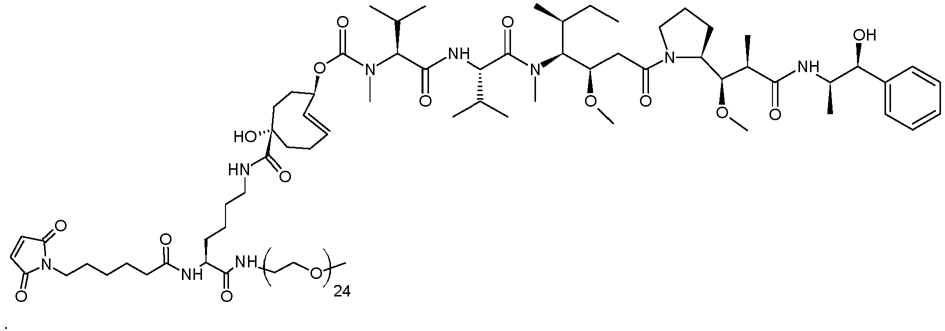

8. The compound according to any one of the preceding claims, wherein said compound is

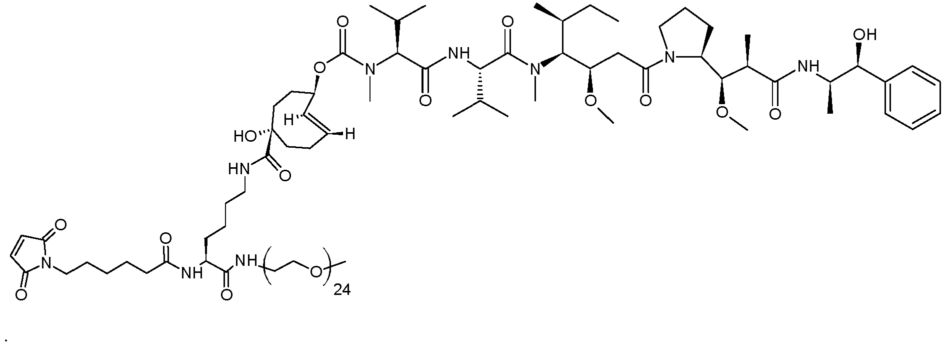

9. A conjugate comprising a protein conjugated to at least one compound according to any one of the preceding claims, wherein said protein and said compound are conjugated via T² and a residue of a sulfhydryl of said protein; wherein the protein is AVP0458 consisting of two monomers, wherein each of the two monomers has an amino acid sequence according to SEQ ID NO: 1; and wherein the protein is conjugated to at most 4 of said compounds.

10. The conjugate according to claim 9, wherein the at least one compound is according to claim 5.

11. The conjugate according to claim 9, wherein the conjugate is 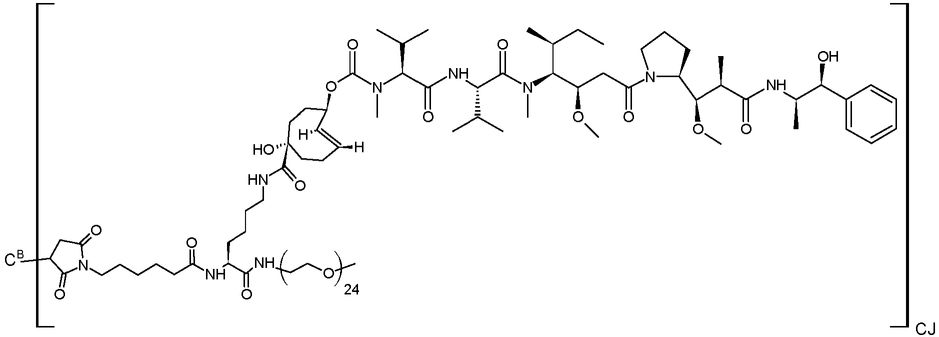 wherein C^{B} is AVP0458; and CJ is of from 3.5 to 4.

12. A composition comprising:
(a) a compound according to any one of claims 1 to 8; and/or
(b) the conjugate according to any one of claims 9 to 11;
preferably the composition is a pharmaceutical composition.

13. A composition according to claim 12, wherein said composition comprises:
(a) a compound according to any one of claims 1 to 6; and
(b) the enantiomer of said compound;
preferably said composition is a racemic mixture of (a) and (b).

14. A combination of
(A1) a compound according to any one of claims 1 to 8;
(A2) a conjugate according to any one of claims 9 to 11; and/or
(A3) a composition according to claim 12 or 13; with
(B) a diene; preferably the diene is a tetrazine.

15. The combination according to claim 14, wherein the diene is selected from the group consisting of: 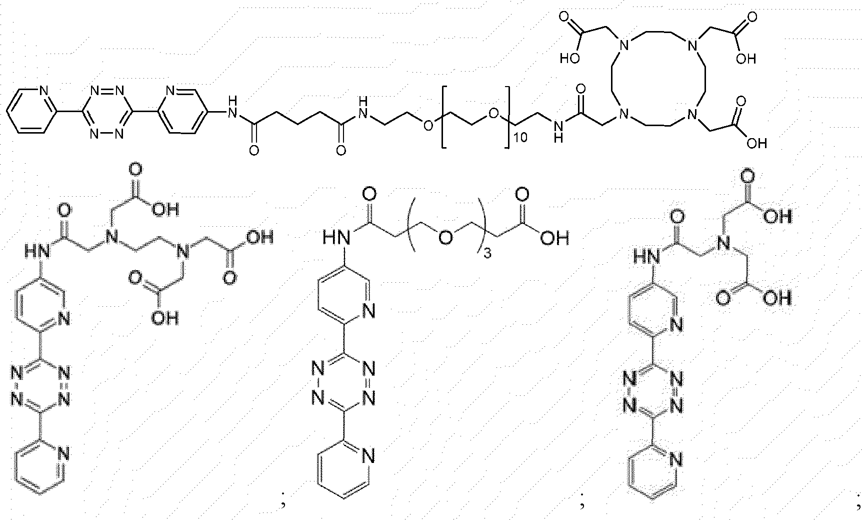 and

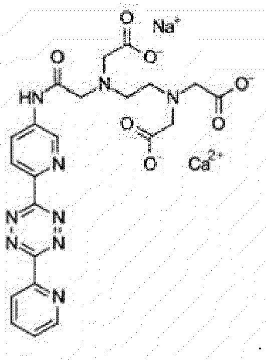

16. The compound according to any one of claims 1 to 8; the conjugate according to any one of claims 9 to 11; the composition according to any one of claims 12 to 13; or the combination according to any one of claims 14 to 15; for use as a medicament.

17. The compound according to any one of claims 1 to 8; the conjugate according to any one of claims 9 to 11; the composition according to any one of claims 12 to 13; or the combination according to any one of claims 14 to 15; for use in the treatment of a disease in a subject; wherein the subject is a human; and the disease is cancer.

18. A non-therapeutic method for reacting:
(ia) the compound according to any one of claims 1 to 8;
(iia) the conjugate according to any one of claims 9 to 11; and/or
(iiia) the composition according to any one of claims 12 to 13;
with a diene,
wherein said method comprises the step of contacting (ia), (iia), or (iiia) with said diene, preferably said non-therapeutic method is an *in vitro* method; and preferably said diene is a tetrazine.

19. A non-therapeutic use of:
(a) the compound according to any one of claims 1 to 8;
(b) the conjugate according to any one of claims 9 to 11;
(c) the composition according to any one of claims 12 to 13; and/or
(d) the combination according to any one of claims 14 to 15;
in a click reaction.

20. A method for synthesizing a compound according to any one of claims 1 to 8; wherein said method comprises
(A) coupling a compound of Formula (R) to a compound of Formula (S): 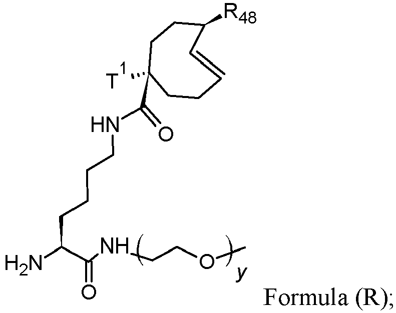 wherein R₄₈, T¹, and y are as defined in any one of claims 1 to 8; 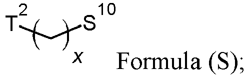 wherein T², and x, are as defined in any one of claims 1 to 8, and S¹⁰ is -COOH or an active ester, preferably S¹⁰ is -COOH;
or
(B) coupling a compound of Formula (T) to a compound of Formula (U): 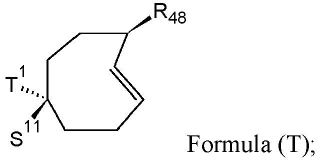 wherein R₄₈, and T¹ are as defined in any one of claims 1 to 8; and S¹¹ is -COOH or an active ester, preferably S¹¹ is an active ester; 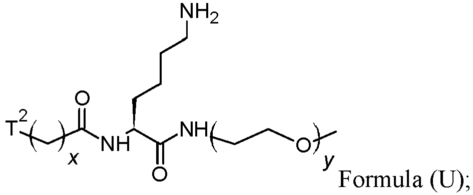 wherein T², x, and y are as defined in any one of claims 1 to 8.

21. A method for synthesizing a conjugate according to any one of claims 9 to 11; wherein said method comprises the step of coupling a protein to a compound according to any one of claims 1 to 8; wherein preferably in said protein disulfide bonds have been reduced.

## Patentansprüche

1. Verbindung gemäß der Formel (2): 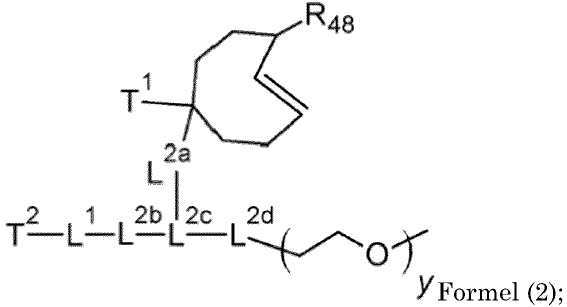 wobei
**L¹** ein lineares C₅-C₆-Alkylen ist;
**T¹**-OH ist;
**R₄₈** -O-C(0)- **C^{A}** ist;
wobei **C^{A}** ein Wirkstoff ist, wobei der Wirkstoff ausgewählt ist aus der Gruppe bestehend aus Monomethyl-Auristatin E, Exatecan und N-Methyl-Exatecan;
wobei der Wirkstoff an die Einheit -O-C(O)- gebunden ist über ein sekundäres oder tertiäres Stickstoffatom, das Teil des Wirkstoffs ist, ein Carbamat bildend;
**L^{2a}**, **L^{2b}** und **L^{2d}** jeweils unabhängig voneinander ausgewählt sind aus der Gruppe bestehend aus -C(O)NH-, und -NHC(O)-;
**L^{2c}** ist C₄-C₆-Alkantriyl;
**T²** ist Maleimidyl;
wobei y eine ganze Zahl im Bereich von 20 bis 30 ist.

2. Verbindung nach Anspruch 1, wobei L¹ ein unsubstituiertes, lineares C₅-Alkylen ist.

3. Verbindung nach einem der vorstehenden Ansprüche, wobei T¹ in einer axialen Position ist und wobei R₄₈ in einer axialen Position ist.

4. Verbindung nach einem der vorstehenden Ansprüche, wobei der Wirkstoff Monomethyl-Auristatin E (MMAE) ist.

5. Verbindung nach einem der vorstehenden Ansprüche, wobei L¹ ein unsubstituiertes, lineares C₅-Alkylen ist, y 24 ist, der Wirkstoff Monomethyl-Auristatin E ist, T¹ in axialer Position ist und R₄₈ in axialer Position ist.

6. Verbindung nach einem der vorstehenden Ansprüche, wobei die Verbindung nach Formel (O) ist: 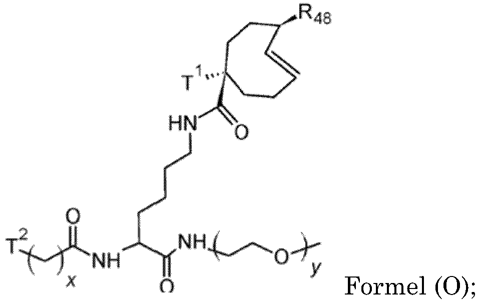 wobei x 5 ist, y 24 ist, der Wirkstoff Monomethyl-Auristatin E ist, T¹ in axialer Position ist und R₄₈ in axialer Position ist.

7. Verbindung nach einem der vorstehenden Ansprüche, wobei die Verbindung ist:

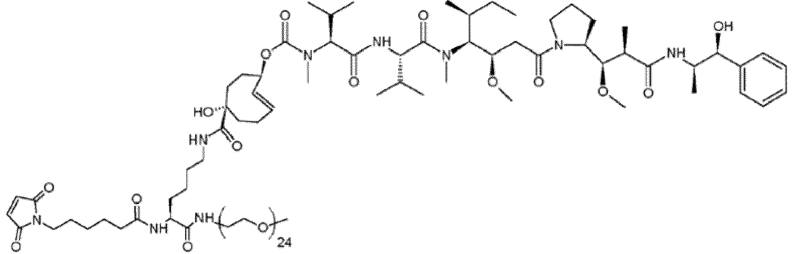

8. Verbindung nach einem der vorstehenden Ansprüche, wobei die Verbindung ist:

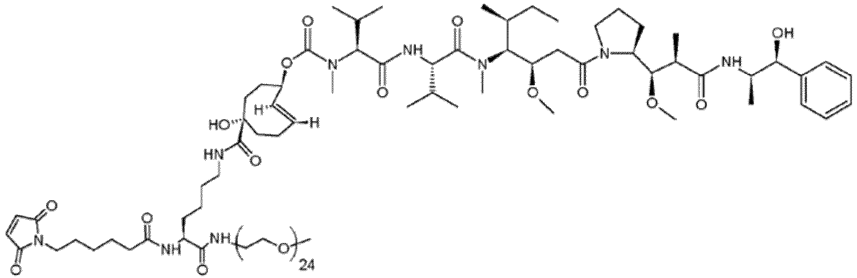

9. Konjugat, umfassend ein Protein, konjugiert an mindestens eine Verbindung nach einem der vorstehenden Ansprüche, wobei das Protein und die Verbindung über T² und einen Sulfhydrylrest des Proteins konjugiert sind; wobei das Protein AVP0458 ist, bestehend aus zwei Monomeren, wobei jedes der zwei Monomere eine Aminosäuresequenz gemäß SEQ ID NR:1 hat; und wobei das Protein an höchstens 4 der Verbindungen konjugiert ist.

10. Konjugat nach Anspruch 9, wobei die mindestens eine Verbindung nach Anspruch 5 ist.

11. Konjugat nach Anspruch 9, wobei das Konjugat 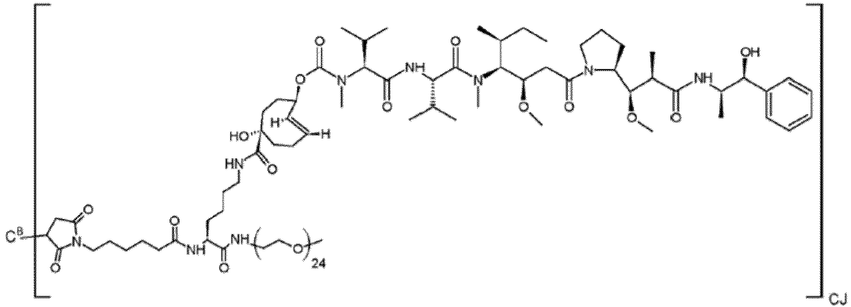 ist, wobei C^{B} AVP0458 ist und CJ von von 3,5 bis 4 ist.

12. Zusammensetzung, umfassend:
(a) eine Verbindung nach einem der Ansprüche 1 bis 8; und/oder
(b) das Konjugat nach einem der Ansprüche 9 bis 11;
wobei vorzugsweise die Zusammensetzung eine pharmazeutische Zusammensetzung ist.

13. Zusammensetzung nach Anspruch 12, wobei die Zusammensetzung umfasst:
(a) eine Verbindung nach einem der Ansprüche 1 bis 6; und
(b) das Enantiomer der Verbindung;
wobei vorzugsweise die Zusammensetzung ein racemisches Gemisch aus (a) und (b) ist.

14. Kombination aus
(A1) einer Verbindung nach einem der Ansprüche 1 bis 8;
(A2) einem Konjugat nach einem der Ansprüche 9 bis 11; und/oder
(A3) einer Zusammensetzung nach Anspruch 12 oder 13; mit
(B) einem Dien; wobei vorzugsweise das Dien ein Tetrazin ist.

15. Kombination nach Anspruch 14, wobei das Dien ausgewählt ist aus der Gruppe bestehend aus: 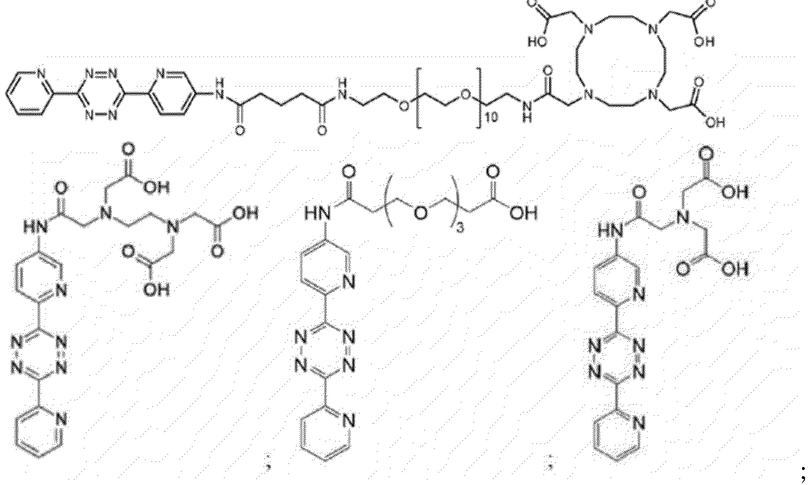 und

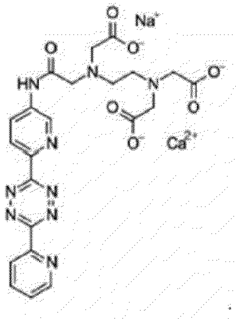

16. Verbindung nach einem der Ansprüche 1 bis 8;
Konjugat nach einem der Ansprüche 9 bis 11;
Zusammensetzung nach einem der Ansprüche 12 bis 13;
oder Kombination nach einem der Ansprüche 14 bis 15;
zur Verwendung als Arzneimittel.

17. Verbindung nach einem der Ansprüche 1 bis 8;
dem Konjugat nach einem der Ansprüche 9 bis 11;
Zusammensetzung nach einem der Ansprüche 12 bis 13;
oder Kombination nach einem der Ansprüche 14 bis 15;
zur Verwendung bei der Behandlung einer Erkrankung eines Subjekts;
wobei das Subjekt ein Mensch ist; und die Erkrankung Krebs ist.

18. Nicht-therapeutisches Verfahren zur Umsetzung:
(ia) der Verbindung nach einem der Ansprüche 1 bis 8;
(iia) des Konjugats nach einem der Ansprüche 9 bis 11; und/oder
(iiia) der Zusammensetzung nach einem der Ansprüche 12 bis 13;
mit einem Dien,
wobei das Verfahren den Schritt des Inkontaktbringens von (ia), (iia) oder (iiia) mit dem Dien umfasst, wobei vorzugsweise das nicht-therapeutische Verfahren ein *In-vitro*-Verfahren ist; und vorzugsweise das Dien ein Tetrazin ist.

19. Nicht-therapeutische Verwendung von:
(a) der Verbindung nach einem der Ansprüche 1 bis 8;
(b) des Konjugats nach einem der Ansprüche 9 bis 11;
(c) der Zusammensetzung nach einem der Ansprüche 12 bis 13; und/oder
(d) der Kombination nach einem der Ansprüche 14 bis 15;
in einer Click-Reaktion.

20. Verfahren zur Synthese einer Verbindung nach einem der Ansprüche 1 bis 8;
wobei das Verfahren umfasst
(A) Koppeln einer Verbindung der Formel (R) an eine Verbindung der Formel (S): 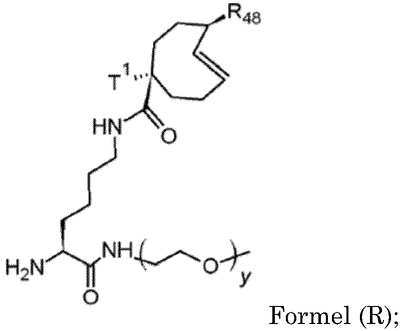 wobei R₄₈, T¹, und y wie in einem der Ansprüche 1 bis 8 definiert sind; 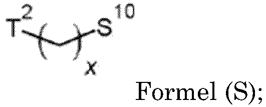 wobei T² und x wie in einem der Ansprüche 1 bis 8 definiert sind und S¹⁰ -COOH oder ein aktiver Ester ist, wobei vorzugsweise S¹⁰ -COOH ist; oder
(B) Koppeln einer Verbindung der Formel (T) an eine Verbindung der Formel (U): 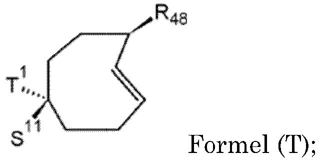
wobei R₄₈, and T¹ wie in einem der Ansprüche 1 bis 8 definiert sind;
und S¹¹ -COOH oder ein aktiver Ester ist, wobei vorzugsweise S¹¹ ein aktiver Ester ist; 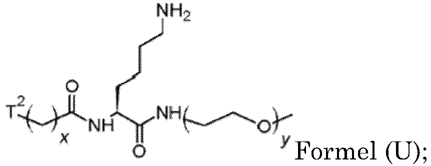
wobei T², x und y wie in einem der Ansprüche 1 bis 8 definiert sind.

21. Verfahren zur Synthese eines Konjugats nach einem der Ansprüche 9 bis 11;
wobei das Verfahren den Schritt des Koppelns eines Proteins an eine Verbindung nach einem der Ansprüche 1 bis 8 umfasst;
wobei vorzugsweise Disulfidbrücken in dem Protein reduziert worden sind.

## Revendications

1. Composé selon la formule (2) : 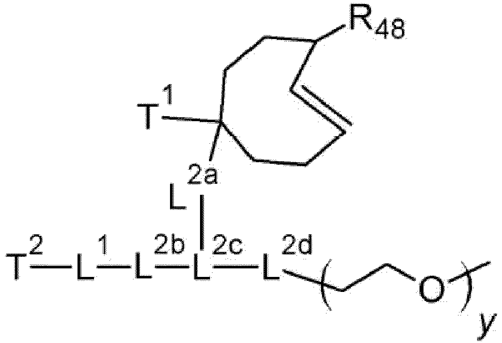
Formule (2) ; dans laquelle
L¹ est un alkylène en C₅-C₆ linéaire ;
T¹ est -OH ;
R₄₈ est -O-C(O)-C^{A} ; où C^{A} est un médicament, où le médicament est choisi dans le groupe consistant en monométhyl auristatine E, exatécan et N-méthyl-exatécan ;
où le médicament est lié à la fraction -O-C(O)- par le biais d'un azote secondaire ou tertiaire qui fait partie du médicament, formant un carbamate ;
L^{2a}, L^{2b} et L^{2d} sont chacun indépendamment choisis dans le groupe consistant en -C(O)NH-, et -NHC(O)- ;
L^{2c} est un alcanetriyle en C₄-C₆ ;
T² est le maléimidyle ;
y est un entier compris dans la plage allant de 20 à 30.

2. Composé selon la revendication 1, dans lequel L¹ est un alkylène en C₅ linéaire non substitué.

3. Composé selon l'une quelconque des revendications précédentes, où T¹ est en une position axiale, et où R₄₈ est en une position axiale.

4. Composé selon l'une quelconque des revendications précédentes, où le médicament est la monométhyl auristatine E (MMAE).

5. Composé selon l'une quelconque des revendications précédentes, dans lequel L¹ est un alkylène en C₅ linéaire non substitué, y vaut 24, le médicament est la monométhyl auristatine E, T¹ est en une position axiale, et R₄₈ est en une position axiale.

6. Composé selon l'une quelconque des revendications précédentes, où ledit composé est conformément à la formule (O) : 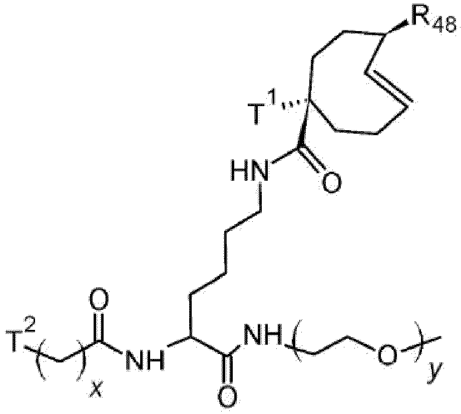 Formule (O) ; dans laquelle x vaut 5, y vaut 24, le médicament est la monométhyl auristatine E, T¹ est en une position axiale, et R₄₈ est en une position axiale.

7. Composé selon l'une quelconque des revendications précédentes, où ledit composé est :

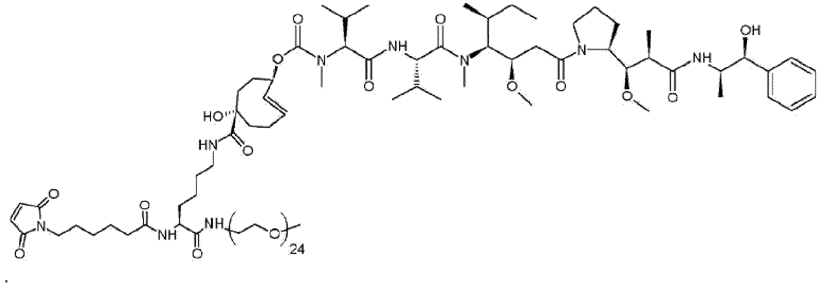

8. Composé selon l'une quelconque des revendications précédentes, où ledit composé est :

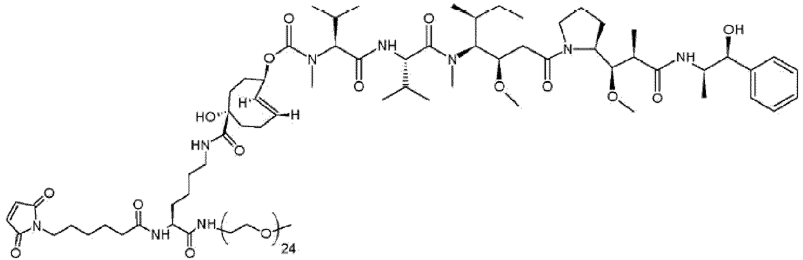

9. Conjugué comprenant une protéine conjuguée à au moins un composé selon l'une quelconque des revendications précédentes, où ladite protéine et ledit composé sont conjugués par l'intermédiaire de T² et un résidu d'un sulfhydryle de ladite protéine ; où la protéine est l'AVP0458 consistant en deux monomères, où chacun des deux monomères a une séquence d'acides aminés selon la SEQ ID NO:1 ; et où la protéine est conjugée à au maximum 4 desdits composés.

10. Conjugué selon la revendication 9, où le au moins un composé est selon la revendication 5.

11. Conjugué selon la revendication 9, où le conjugué est 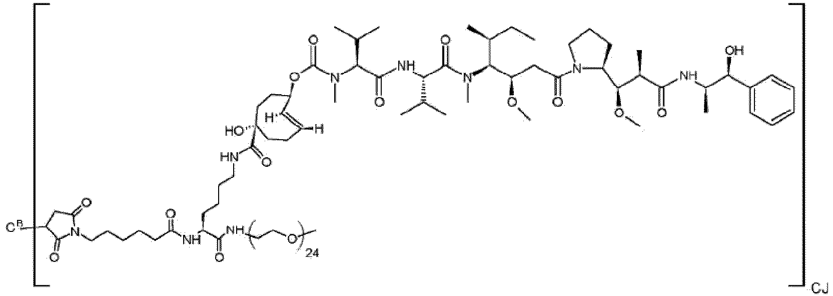 où C^{B} est AVP0458 ; et CJ vaut de 3,5 à 4.

12. Composition comprenant :
(a) un composé selon l'une quelconque des revendications 1 à 8 ; et/ou
(b) le conjugué selon l'une quelconque des revendications 9 à 11 ;
de préférence la composition est une composition pharmaceutique.

13. Composition selon la revendication 12, où ladite composition comprend :
(a) un composé selon l'une quelconque des revendications 1 à 6 ; et
(b) l'énantiomère dudit composé ;
de préférence ladite composition est un mélange racémique de (a) et (b).

14. Combinaison de
(A1) un composé selon l'une quelconque des revendications 1 à 8 ;
(A2) un conjugué selon l'une quelconque des revendications 9 à 11 ; et/ou
(A3) une composition selon la revendication 12 ou 13 ; avec
(B) un diène ; de préférence le diène est une tétrazine.

15. Combinaison selon la revendication 14, dans laquelle le diène est choisi dans le groupe consistant en : 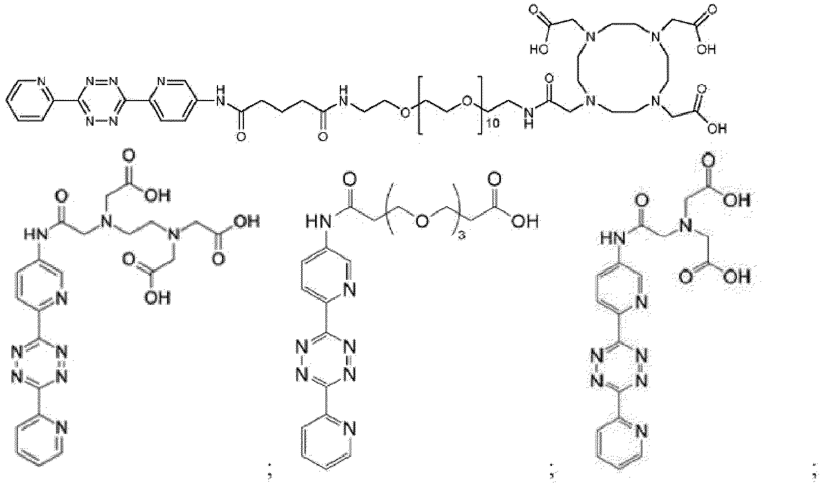 et

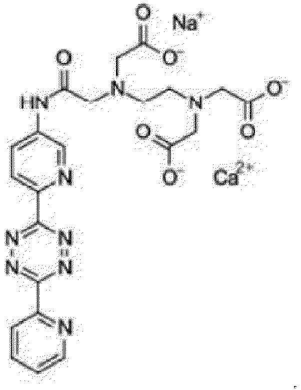

16. Composé selon l'une quelconque des revendications 1 à 8 ; conjugué selon l'une quelconque des revendications 9 à 11 ; composition selon l'une quelconque des revendications 12 à 13 ; ou combinaison selon l'une quelconque des revendications 14 à 15 ; pour l'utilisation comme médicament.

17. Composé selon l'une quelconque des revendications 1 à 8 ; conjugué selon l'une quelconque des revendications 9 à 11 ; composition selon l'une quelconque des revendications 12 à 13 ; ou combinaison selon l'une quelconque des revendications 14 à 15 ; pour l'utilisation dans le traitement d'une maladie chez un sujet ; où le sujet est un humain ; et la maladie est le cancer.

18. Procédé non thérapeutique pour faire réagir :
(ia) le composé selon l'une quelconque des revendications 1 à 8 ;
(iia) le conjugué selon l'une quelconque des revendications 9 à 11 ; et/ou
(iiia) la composition selon l'une quelconque des revendications 12 à 13 ;
avec un diène,
où ledit procédé comprend l'étape de mise en contact de (ia), (iia) ou (iiia) avec ledit diène, de préférence ledit procédé non thérapeutique est un procédé in vitro ; et de préférence ledit diène est une tétrazine.

19. Utilisation non thérapeutique :
(a) du composé selon l'une quelconque des revendications 1 à 8 ;
(b) du conjugué selon l'une quelconque des revendications 9 à 11 ;
(c) de la composition selon l'une quelconque des revendications 12 à 13 ; et/ou
(d) de la combinaison selon l'une quelconque des revendications 14 à 15 ;
dans une réaction click.

20. Procédé de synthèse d'un composé selon l'une quelconque des revendications 1 à 8 ; où ledit procédé comprend
(A) le couplage d'un composé de formule (R) à un composé de formule (S) : 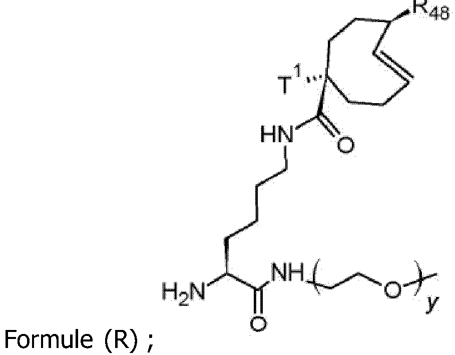 dans laquelle R₄₈, T¹ et y sont tels que définis selon l'une quelconque des revendications 1 à 8 ; 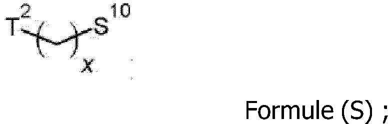 dans laquelle T² et x sont tels que définis selon l'une quelconque des revendications 1 à 8, et S¹⁰ est - COOH ou un ester actif, de préférence S¹⁰ est -COOH ;
ou
(B) le couplage d'un composé de formule (T) à un composé de formule (U) : 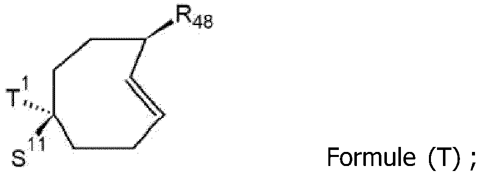 **dans laquelle** R₄₈ et T¹ sont tels que définis selon l'une quelconque des revendications 1 à 8 ; et S¹¹ est -COOH ou un ester actif, de préférence S¹¹ est un ester actif ; 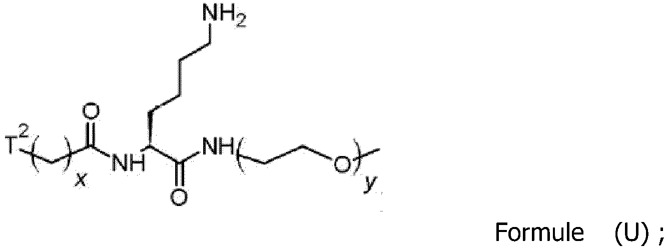 dans laquelle T², x et y sont tels que définis selon l'une quelconque des revendications 1 à 8.

21. Procédé de synthèse d'un conjugué selon l'une quelconque des revendications 9 à 11 ; où ledit procédé comprend l'étape de couplage d'une protéine à un composé selon l'une quelconque des revendications 1 à 8 ; où de préférence, dans ladite protéine, les liaisons disulfure ont été réduites.
